(19) Europäisches Patentamt / European Patent Office / Office européen des brevets

(11) **EP 3 677 914 A1**

(12) **EUROPEAN PATENT APPLICATION**

(43) Date of publication:
08.07.2020 Bulletin 2020/28

(21) Application number: 20158817.5

(22) Date of filing: 31.10.2016

(51) Int Cl.:
*G01N 33/68* (2006.01)        *G01N 24/08* (2006.01)
*G01N 33/49* (2006.01)        *H01J 49/40* (2006.01)
*G16H 50/20* (2018.01)        *G16H 10/40* (2018.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 12.11.2015  US 201562254185 P

(62) Document number(s) of the earlier application(s) in accordance with Art. 76 EPC:
16864066.2 / 3 376 229

(71) Applicants:
• **Kyushu University National University Corporation**
Fukuoka 812-8581 (JP)
• **National Center of Neurology and Psychiatry Kodaira-shi**
Tokyo 187-8551 (JP)

(72) Inventors:
• **KATO, Takahiro**
FUKUOKA-SHI FUKUOKA, 812-8581 (JP)
• **SETOYAMA, Daiki**
FUKUOKA-SHI, FUKUOKA, 812-8581 (JP)
• **KANG, Dongchon**
FUKUOKA-SHI, FUKUOKA, 812-8581 (JP)
• **KANBA, Shigenobu**
FUKUOKA-SHI, FUKUOKA, 812-8581 (JP)
• **HASHIMOTO, Ryota**
SUITA-SHI, OSAKA, 565-0871 (JP)
• **KUNUGI, Hiroshi**
KODAIRA-SHI, TOKYO, 187-8551 (JP)
• **HATTORI, Kotaro**
KODAIRA-SHI, TOKYO, 187-8551 (JP)

(74) Representative: **EP&C**
P.O. Box 3241
2280 GE Rijswijk (NL)

Remarks:
This application was filed on 21.02.2020 as a divisional application to the application mentioned under INID code 62.

(54) **BIOMARKER FOR DIAGNOSING DEPRESSION AND USE OF BIOMARKER**

(57) The present invention provides an objective biomarker which is capable of evaluating the severity of depression and is clinically useful. The present invention provides a biomarker for evaluating the severity of depression, including at least one compound selected from the group consisting of 4-aminobutyric acid (y (gamma)-aminobutyric acid: GABA), arginine, argininosuccinate, isoleucine, indole carboxaldehyde, potassium indoleacetate, carnitine, acetylcarnitine, ornithine, xanthurenate, kynurenate, kynurenine, citrate, creatine, creatinine, glutamine, dimethylglycine, serotonin, taurine, trimethyloxamine (TMAO), tryptophan, norvaline, 3-hydroxybutyrate, phenylalanine, proline, betaine, and lysine.

EP 3 677 914 A1

## Description

Technical Field

The present invention relates to a biomarker for diagnosing depression and the use of the biomarker.
Priority is claimed on US Provisional Patent Application No. 62/254,185 filed on November 12, 2015, the content of which is incorporated herein by reference.

Background Art

Depression has various symptoms such as feelings of guilt and suicidal ideation in addition to depressive feelings and loss of interest and is an illness carrying the highest risk of suicide, and therefore the establishment of a method for evaluating the severity of depression is an urgent priority. It is possible to evaluate the severity of depression to some extent with a self-administered questionnaire such as Patient Health Questionnaire (PHQ)-9, or a semi-structured interview by an expert such as the Hamilton Rating Scale for Depression (HAMD), but the evaluation is dependent on a subjective complaint or behavior of a patient.
PTLS 1 to 3 disclose biomarkers for objectively diagnosing depression.

Citation List

Patent Literature

[PTL 1] Republished Japanese Translation No. WO2011/019072 of the PCT International Publication for Patent Applications
[PTL 2] Japanese Unexamined Patent Application, First Publication No. 2014-013257
[PTL 3] Japanese Patent No. 5372213

Summary of Invention

Technical Problem

The biomarkers for depression disclosed in PTLS 1 to 3 are capable of diagnosing whether depression has occurred or not but are not capable of accurately evaluating the severity of depression. Accordingly, an objective biomarker which is capable of evaluating the severity of depression and is clinically useful has been required.
The present invention has been made in view of the above circumstances and provides an objective biomarker which is capable of evaluating the severity of depression and is clinically useful.

Solution to Problem

The inventors of the present invention have conducted intensive studies to achieve the above object, and as a result, have identified a plurality of metabolites contributing to the severity of depression, have found that by metabolomic analysis using a blood sample of a patient with depression, the metabolites contributing to each of various depression symptoms such as depressive feelings, loss of interest, suicidal ideation, and feelings of guilt are different, and therefore have completed the present invention.
That is, the present invention includes the following aspects.

[1] A biomarker for evaluating the severity of depression, including at least one compound selected from the group consisting of 4-aminobutyric acid ($\gamma$ (gamma)-aminobutyric acid: GABA), arginine, argininosuccinate, isoleucine, indole carboxaldehyde, potassium indoleacetate, carnitine, acetylcarnitine, ornithine, xanthurenate, kynurenate, kynurenine, citrate, creatine, creatinine, glutamine, dimethylglycine, serotonin, taurine, trimethyloxamine (TMAO), tryptophan, norvaline, 3-hydroxybutyrate, phenylalanine, proline, betaine, and lysine.
[2] The biomarker according to [1], further including at least one compound selected from the group consisting of cholesterol, uric acid, bilirubin, and cytokines.
[3] A method for evaluating the severity of depression, including: a measurement step of measuring blood concentrations of biomarkers according to [1] or [2] of a subject; a discriminant value calculation step of calculating a discriminant value which is a value of a multivariate discriminant based on a blood concentration of at least one biomarker of the blood concentrations of the biomarkers measured in the measurement step and the multivariate discriminant set in advance which has the blood concentration of the biomarker as a variable and has at least one biomarker as the variable; and an evaluation step of evaluating the severity of depression of the subject to be tested based on the discriminant value calculated in the discriminant value calculation step.

[4] The method for evaluating the severity of depression according to [3], in which the multivariate discriminant is one fractional expression, a sum of a plurality of the fractional expressions, a logistic regression equation, a linear discriminant, a multiple regression equation, an equation created with a support vector machine, an equation created by the Mahalanobis distance method, an equation created by canonical discriminant analysis, or an equation created with a decision tree.

[5] A program for evaluating the severity of depression which causes a computer to execute: an acquisition step of acquiring concentrations of the biomarkers according to [1] or [2] in blood collected from a subject to be tested; a discriminant value calculation step of calculating a discriminant value which is a value of a multivariate discriminant based on a blood concentration of at least one biomarker of the blood concentrations of the biomarkers acquired in the acquisition step and the multivariate discriminant set in advance which has the blood concentration of the biomarker as a variable and has at least one biomarker as the variable; an evaluation step of evaluating the severity of depression of the subject to be tested based on the discriminant value calculated in the discriminant value calculation step; and an output step of outputting the obtained evaluation results.

[6] The program for evaluating the severity of depression according to [5], which causes a computer to further execute: a step of causing a biomarker-measuring apparatus to measure the concentrations of the biomarkers in blood before the acquisition step.

[7] A biomarker for predicting loss of interest/pleasure of a subject to be tested in at least any one of Patient Health Questionnaire (PHQ)-9, Beck Depression Inventory-II (BDI-2), and the Hamilton Rating Scale for Depression (HAMD), the biomarker including at least one compound selected from the group consisting of acetylcarnitine, urocanic acid, 2-oxobutanoate, carbamoyl phosphate, proline, and 3-methylhistidine.

[8] A method for predicting loss of interest/pleasure of a subject to be tested in at least any one of PHQ-9, BDI-2, and HAMD, the method including using a blood concentration of at least one compound selected from the group consisting of acetylcarnitine, urocanic acid, 2-oxobutanoate, carbamoyl phosphate, proline, and 3-methylhistidine of the subject to be tested.

[9] A biomarker for predicting depressive feelings of a subject to be tested in at least any one of PHQ-9, BDI-2, and HAMD, the biomarker including at least one compound selected from the group consisting of N-acetylglutamate, 2-oxobutanoate, carnosine, 5-hydroxytryptophan, proline, and melatonin.

[10] A method for predicting depressive feelings of a subject to be tested in at least any one of PHQ-9, BDI-2, and HAMD, the method including using a blood concentration of at least one compound selected from the group consisting of N-acetylglutamate, 2-oxobutanoate, carnosine, 5-hydroxytryptophan, proline, and melatonin of the subject to be tested.

[11] A biomarker for predicting feelings of worthlessness/guilt of a subject to be tested in at least any one of PHQ-9, BDI-2, and HAMD, the biomarker including at least one compound selected from the group consisting of agmatine, adenosine triphosphate (ATP), argininosuccinate, tryptophan, valine, 5-hydroxytryptophan, proline, and phosphoenolpyruvate.

[12] A method for predicting feelings of worthlessness/guilt of a subject to be tested in at least any one of PHQ-9, BDI-2, and HAMD, the method including using a blood concentration of at least one compound selected from the group consisting of agmatine, ATP, argininosuccinate, tryptophan, valine, 5-hydroxytryptophan, proline, and phosphoenolpyruvate of the subject to be tested.

[13] A biomarker for predicting agitation/retardation of a subject to be tested in at least any one of PHQ-9, BDI-2, and HAMD, the biomarker including at least one compound selected from the group consisting of citrate, creatine, 5-hydroxytryptophan, 4-hydroxyproline, 3-hydroxybutyrate, fumarate, proline, and leucine.

[14] A method for predicting agitation/retardation of a subject to be tested in at least any one of PHQ-9, BDI-2, and HAMD, the method including using a blood concentration of at least one compound selected from the group consisting of citrate, creatine, 5-hydroxytryptophan, 4-hydroxyproline, 3-hydroxybutyrate, fumarate, proline, and leucine of the subject to be tested.

[15] A biomarker for predicting suicidal ideation of a subject to be tested in at least any one of PHQ-9, BDI-2, and HAMD, the biomarker including at least one compound selected from the group consisting of N-acetylglutamate, alanine, xanthurenate, xanthosine, kynurenine, kynurenate, citrate, 3-hydroxykynurenine, phenylalanine, and phosphoenolpyruvate.

[16] A method for predicting suicidal ideation of a subject to be tested in at least any one of PHQ-9, BDI-2, and HAMD, the method including using a blood concentration of at least one compound selected from the group consisting of N-acetylglutamate, alanine, xanthurenate, xanthosine, kynurenine, kynurenate, citrate, 3-hydroxykynurenine, phenylalanine, and phosphoenolpyruvate of the subject to be tested.

[17] A biomarker for predicting sleep-related disorder of a subject to be tested in at least any one of PHQ-9, BDI-2, and HAMD, the biomarker including at least one compound selected from the group consisting of agmatine, N-acetylaspartate, N-acetylglutamine, adenine, adenosine monophosphate (AMP), ATP, isocitrate, ornithine, carnitine, citrate, glucosamine, β-glycerophosphate, serotonin, tyrosine, threonine, pyruvate, pyroglutamate, phenylalanine, fumarate, pantothenate, 2-phosphoglycerate, 5-phosphoribosyl-1-pyrophosphate (PRPP), methionine, and 3-methylhistidine.

[18] A method for predicting sleep-related disorder of a subject to be tested in at least any one of PHQ-9, BDI-2, and HAMD, the method including using a blood concentration of at least one compound selected from the group consisting of agmatine, N-acetylaspartate, N-acetylglutamine, adenine, AMP, ATP, isocitrate, ornithine, carnitine, citrate, glucosamine, β-glycerophosphate, serotonin, tyrosine, threonine, pyruvate, pyroglutamate, phenylalanine, fumarate, pantothenate, 2-phosphoglycerate, PRPP, methionine, and 3-methylhistidine.

[19] A biomarker for predicting fatigue of a subject to be tested in at least any one of PHQ-9, BDI-2, and HAMD, the biomarker including at least one compound selected from the group consisting of asparagine, N-acetylaspartate, 2-oxobutyrate, ornithine, β-glycerophosphate, melatonin, and proline.

[20] A method for predicting fatigue of a subject to be tested in at least any one of PHQ-9, BDI-2, and HAMD, the method including using a blood concentration of at least one compound selected from the group consisting of asparagine, N-acetylaspartate, 2-oxobutyrate, ornithine, β-glycerophosphate, melatonin, and proline.

Advantageous Effects of Invention

According to the present invention, it is possible to provide an objective biomarker which is capable of evaluating the severity of depression and is clinically useful.

Brief Description of Drawings

FIG. 1 is a diagram showing a selection flow of a plasma sample of a patient with depression in Kyushu University, Osaka University, and National Center of Neurology and Psychiatry in Example 1.

FIG. 2A is a graph showing the relationship between measurement values of scores of PHQ-9 and predictive values obtained by a regression model for predicting scores of PHQ-9 in a data set-1 of Example 1.

FIG. 2B is a graph showing the relationship between measurement values of scores of HAMD-17 and predictive values obtained by a regression model for predicting scores of HAMD-17 in the data set-1 of Example 1.

FIG. 2C is a graph showing the relationship between measurement values of scores of HAMD-17 and predictive values obtained by a regression model for predicting scores of HAMD-17 in a data set-2 of Example 1.

FIG. 2D is a graph showing the relationship between measurement values of scores of HAMD-17 and predictive values obtained by a regression model for predicting scores of HAMD-17 in a data set-3 of Example 1.

FIG. 3 is a table showing metabolites having a high degree of contribution (VIP > 1.0) to predictive values obtained by a regression model for predicting scores of PHQ-9 or HAMD-17 in the data sets-1 to 3 of Example 1, in the order of a higher degree of contribution.

FIG. 4A is a table showing metabolites having a moderate correlation (an absolute value of a correlation coefficient is 0.3 or more) with various symptoms of PHQ-9 or HAMD-17 in the data set-1 of Example 1.

FIG. 4B is a table showing metabolites having a moderate correlation (an absolute value of a correlation coefficient is 0.3 or more) with sleep disorders or fatigue of PHQ-9 or HAMD-17 in the data set-1 of Example 1.

FIG. 5 is a diagram showing a correlation network between subscales (various symptoms of depression) of HAMD-17 in the data set-1 and metabolites of Example 1.

FIG. 6 is a table showing metabolites having a moderate correlation with SI of HAMD-17 in all the data sets-1 to 3 of Example 1.

FIG. 7A is a graph showing receiver operating characteristic (ROC) curves derived from ten types of logistic regression models which are for predicting suicide attempts of HAMD-17 of a patient with depression in Example 1.

FIG. 7B is a graph showing a significant correlation (R = 0.22, p = 0.028) between suicide attempts of HAMD-17 and predictive values obtained by a regression model using a multiple linear discriminant having variables of citrate and kynurenine in plasma, of which intensity has been standardized, in Example 1.

FIG. 8 is a graph showing the relationship between measurement values of scores of BDI-II and predictive values obtained by a regression model for predicting scores of BDI-II in Example 2.

Description of Embodiments

<Biomarker for Evaluating Severity of Depression>

In one embodiment, the present invention provides a biomarker for evaluating the severity of depression, including at least one compound selected from the group consisting of 4-aminobutyric acid ($\gamma$ (gamma)-aminobutyric acid: GABA), arginine, argininosuccinate, isoleucine, indole carboxaldehyde, potassium indoleacetate, carnitine, acetylcarnitine, ornithine, xanthurenate, kynurenate, kynurenine, citrate, creatine, creatinine, glutamine, dimethylglycine, serotonin, taurine, trimethyloxamine (TMAO), tryptophan, norvaline, 3-hydroxybutyrate, phenylalanine, proline, betaine, and lysine.

According to the biomarkers of the present embodiment, it is possible to easily and accurately evaluate the severity of depression. In addition, based on the biomarkers of the present embodiment, the present invention can be applied to elucidation of the pathophysiological mechanism of depression.

The biomarker for evaluating the severity of depression of the present embodiment, includes at least one compound selected from the group consisting of 4-aminobutyric acid ($\gamma$ (gamma)-aminobutyric acid: GABA), arginine, argininosuccinate, isoleucine, indole carboxaldehyde, potassium indoleacetate, carnitine, acetylcarnitine, ornithine, xanthurenate, kynurenate, kynurenine, citrate, creatine, creatinine, glutamine, dimethylglycine, serotonin, taurine, trimethyloxamine (TMAO), tryptophan, norvaline, 3-hydroxybutyrate, phenylalanine, proline, betaine, and lysine.

The biomarker for evaluating the severity of depression of the present embodiment, preferably further includes at least one compound selected from the group consisting of cholesterol, uric acid, bilirubin, and cytokines.

Examples of the cholesterol include low-density lipoprotein (LDL) cholesterol, high-density lipoprotein (HDL) cholesterol, and the like.

Examples of the cytokines include, but are not limited to, interleukin-1$\beta$, interleukin-4, interleukin-6, interleukin-10, interleukin-12, tumor necrosis factor-$\alpha$ (TNF-$\alpha$), and the like.

<Method for Evaluating Severity of Depression>

In one embodiment, the present invention provides a method for evaluating the severity of depression, including: a measurement step of measuring the blood concentrations of the biomarkers of a subject to be tested; a discriminant value calculation step of calculating a discriminant value which is a value of a multivariate discriminant based on a blood concentration of at least one biomarker of the blood concentrations of the biomarkers measured in the measurement step and the multivariate discriminant set in advance which has the blood concentration of the biomarker as a variable and has at least one biomarker as the variable; and an evaluation step of evaluating the severity of depression of the subject to be tested based on the discriminant value calculated in the discriminant value calculation step.

According to the evaluation method of the present embodiment, it is possible to easily and accurately evaluate the severity of depression.

In the present specification, ages of subjects to be tested are not limited, and for example, a subject to be tested may be a young subject, specifically a 30-year-old or younger subject, or may be an elderly subject, specifically a subject over 65 years old.

Each step of the evaluation method of the present embodiment will be described in detail below.

[Measurement Step] First, concentrations of the above-described biomarkers contained in the blood collected from the subject to be tested are measured.

It is preferable to measure at least two or more compounds of the above-described biomarkers, it is more preferable to measure all compounds of 4-aminobutyric acid ($\gamma$ (gamma)-aminobutyric acid: GABA), arginine, argininosuccinate, isoleucine, indole carboxaldehyde, potassium indoleacetate, carnitine, acetylcarnitine, ornithine, xanthurenate, kynurenate, kynurenine, citrate, creatine, creatinine, glutamine, dimethylglycine, serotonin, taurine, trimethyloxamine (TMAO), tryptophan, norvaline, 3-hydroxybutyrate, phenylalanine, proline, betaine, and lysine, and it is even more preferable to measure all compounds of 4-aminobutyric acid ($\gamma$ (gamma)-aminobutyric acid: GABA), arginine, argininosuccinate, isoleucine, indole carboxaldehyde, potassium indoleacetate, carnitine, acetylcarnitine, ornithine, xanthurenate, kynurenate, kynurenine, citrate, creatine, creatinine, glutamine, dimethylglycine, serotonin, taurine, trimethyloxamine (TMAO), tryptophan, norvaline, 3-hydroxybutyrate, phenylalanine, proline, betaine, lysine, cholesterol, uric acid, bilirubin, and cytokines.

By combining a plurality of biomarkers in combination at the time of measurement, it is possible to improve the accuracy of evaluating the severity of depression.

Blood used in the present embodiment includes not only blood collected from the subject to be tested but also blood obtained by processing the collected blood. The blood obtained by processing the collected blood includes, for example, serum, plasma, and the like. Serum and plasma are obtained by, for example, allowing blood to stand still or be centrifuged.

In the present specification, blood, serum, and plasma may be collectively referred to as a "blood sample" in some cases.

The blood sample may be used for measuring the concentrations of the biomarkers as it is but may be used for measuring the concentrations of the biomarkers after being subjected to an appropriate preprocessing as necessary. Examples of the preprocessing include stopping enzymatic reactions in the blood sample, removing lipid-soluble substances, removing proteins, and the like. These preprocessings may be carried out by using a known method.

In addition, the blood sample may be diluted or concentrated as appropriate before use.

As the method for measuring the concentrations of the biomarkers, a known method may be appropriately selected according to the types of various biomarkers.

For example, the concentrations of the biomarkers can be measured by selecting a quantification method, according to the marker to be measured, from quantification by nuclear magnetic resonance (NMR), quantification by neutralization titration, quantification by amino acid analyzer, quantification by enzyme method, quantification using aptamers such as nucleic acid aptamers and peptide aptamers, colorimetric determination, and the like, and utilizing the quantification method.

In addition, the concentrations of the biomarkers can also be measured using a commercially available quantification kit according to the biomarker to be measured.

Furthermore, for example, capillary electrophoresis, liquid chromatography, gas chromatography, mass spectrometry, and the like may be used alone or in appropriate combination so that the concentrations of the biomarkers can be measured. These measurement methods are particularly suitable when collectively measuring the plurality of biomarkers.

Examples of a measurement method suitable for highly ionic biomarkers include measurement by capillary electrophoresis-mass spectrometry, and the like. Specifically, the concentrations of the biomarkers can be measured with a capillary electrophoresis-time-of-flight mass spectrometry (CE-TOFMS), for example.

A capillary for the capillary electrophoresis is preferably a fused-silica capillary. In addition, the inner diameter of the capillary may be, for example, 100 $\mu$m or less, and, for example, 50 $\mu$m or less, in consideration of improvement in separability. The total length of the capillary may be, for example, 50 cm to 150 cm.

A method for identifying a fraction containing the compound which is the above-described target biomarker in each fraction obtained by the above-described capillary electrophoresis is not particularly limited, and examples of the method include a method for measuring an electrophoresis time for a compound in advance by using a sample of the target compound, or a method in which a time relative to an electrophoresis time for internal standard substances is used, and the like.

Subsequently, the content of the compound having m/z of the target compound in the fraction identified as containing the compound which is the above-described target biomarker is measured as the peak surface area. The peak surface area can be normalized by taking a ratio to the peak surface area of internal standard substances. In addition, by creating a calibration curve using a sample of the target compound, an absolute concentration of the compound which is the above-described target biomarker contained in collected blood can be obtained from the measured peak surface area. The calibration curve is preferably created not by a standard solution method but by a standard addition method.

In addition, the blood sample used for the measurement using CE-TOFMS may contain an internal standard substance as a measurement standard of an electrophoresis time and a content of the compound which is the above-described biomarker.

The internal standard substance is not particularly limited as long as the substance does not affect the efficiency of electrophoresis-mass spectrometry of the compound which is the above-described biomarker, and examples thereof include methionine sulfone, 10-camphorsulfonic acid (CSA), and the like.

[Discriminant Value Calculation Step] Subsequently, a discriminant value which is a value of a multivariate discriminant is calculated based on a blood concentration of at least one biomarker of the measured blood concentrations of the biomarkers and the multivariate discriminant set in advance which has the blood concentration of the biomarker as a variable and has at least one biomarker as the variable.

Before calculating the discriminant value, data such as a missing value or an outlier may be removed from data of the measured blood concentrations of the biomarkers. Therefore, the severity of depression can be evaluated more accurately.

The multivariate discriminant may be one fractional expression, a sum of a plurality of the fractional expressions, a logistic regression equation, a linear discriminant, a multiple regression equation, an equation created with a support vector machine, an equation created by the Mahalanobis distance method, an equation created by canonical discriminant analysis, or an equation created with a decision tree.

Specifically, the multivariate discriminant may be a linear discriminant expressed by Equation [1] (a linear discriminant with the biomarker as a variable) or a logistic regression equation with the biomarker as a variable.

$$Y = A_1X1 + A_2X2 + A_3X3 + A_4X4 + ... + A_mXn ......[1]$$

(In Equation [1], Y represents a predictive value of the severity of depression, $A_1$, $A_2$, $A_3$, $A_4$, and $A_n$ each independently represents coefficients and are arbitrary real numbers. X1, X2, X3, X4, and Xn each independently represents the blood concentrations of the biomarkers. In addition, m and n are arbitrary integers.)

By using the discriminant value calculated by applying the blood concentrations of various biomarkers measured in the measurement step as the multivariate discriminant, it is possible to more accurately discriminate the severity of depression.

In addition, in the present specification, the term "fractional expression" means that a numerator of the fractional expression is represented by a sum of the biomarkers X1, X2, X3...Xn and/or a denominator of the fractional expression is represented by a sum of the biomarkers x1, x2, x3...xn.

In addition, the fractional expression includes a sum of such fractional expressions α, β, γ,... (such as α + β).

Furthermore, the fractional expression includes a divided fractional expression.

The biomarkers used for the numerator and the denominator may each have an appropriate coefficient.

In addition, the biomarkers used for the numerator and the denominator may overlap.

Furthermore, an appropriate coefficient may be included in each fraction expression.

Furthermore, a value of the coefficient of each variable and a value of a constant term may be real numbers.

In a combination in which a numerator variable and a denominator variable are exchanged in the fractional expression, positive and negative signs of correlation with a target variable generally reverse, but the correlation between the signs and the variable is maintained. Therefore, the discrimination can be regarded as equivalent, and thus the combination in which the numerator variable and the denominator variable are exchanged is also included.

In addition, in the present specification, the term "multivariate discriminant" generally means a form of an expression used in multivariate analysis. Examples of the multivariate discriminant include, but are not limited to, a multiple regression equation, a multiple logistic regression equation, a linear discriminant function, Mahalanobis distance, a canonical discriminant function, a support vector machine, a decision tree, and the like.

In addition, the multivariate discriminant includes an expression as shown by a sum of multivariate discriminants of different types.

Furthermore, in the multiple regression equation, the multiple logistic regression equation, and the canonical discriminant function, coefficients and constant terms are added to each variable, but in this case, the coefficients and constant terms are preferably real numbers, it is more preferable that a value belong to the range of the 99% confidence interval of the coefficients and constant terms obtained from data for discrimination, and it is even more preferable that a value belong to the range of the 95% confidence interval of the coefficients and constant terms obtained from data for discrimination.

Furthermore, the value of each coefficient and the confidence interval may be multiplied by a real number, and the value of the constant term and the confidence interval thereof may be obtained by adding or subtracting an arbitrary real constant.

When expressions such as logistic regression, linear discrimination, and multiple regression analysis are used as indices, because in the linear transformation of the expression (addition of constants, constant multiplication) and monotonic increase (decrease) transformation (for example, logit transform and the like), the discrimination performance is not changed and equivalent, and thus are included in the expression.

In addition, as the biomarker used in the multivariate discriminant, it is preferable to use the combination of a blood concentration of at least one compound selected from the group (hereinafter, will be referred to as "first biomarker group" in some cases) consisting of 4-aminobutyric acid (γ (gamma)-aminobutyric acid: GABA), arginine, argininosuccinate, isoleucine, indole carboxaldehyde, potassium indoleacetate, carnitine, acetylcarnitine, ornithine, xanthurenate, kynurenate, kynurenine, citrate, creatine, creatinine, glutamine, dimethylglycine, serotonin, taurine, trimethyloxamine (TMAO), tryptophan, norvaline, 3-hydroxybutyrate, phenylalanine, proline, betaine, and lysine, and a blood concentration of at least one compound selected from the group (hereinafter, will be referred to as "second biomarker group" in some cases) consisting of cholesterol, uric acid, bilirubin, and cytokines. It is more preferable to use the combination of blood concentrations of two or more compounds selected from the first biomarker group and blood concentrations of two or more compounds selected from the second biomarker group. It is even more preferable to use the combination of blood concentrations of all compounds of the first biomarker group and blood concentrations of all compounds of the second biomarker group.

By incorporating the plurality of biomarkers in the multivariate discriminant, it is possible to improve the accuracy of evaluating the severity of depression.

In addition, as a variable in the multivariate discriminant, biological information of other subjects to be tested (for example, biological metabolites such as minerals and hormones; gender, age, eating habits, drinking habits, fitness habits, degree of obesity, history of disease, interview data, and the like) may be further used, in addition to the biomarkers.

[Evaluation Step] Subsequently, the severity of depression of the subject to be tested is evaluated based on the discriminant value calculated in the discriminant value calculation step.

Specifically, by comparing the discriminant value with a predetermined threshold value (cut-off value), it is possible to discriminate whether a patient is in a group of patients with depression or a group of patients not having depression (healthy subject group).

Furthermore, it is possible to evaluate that the severity of depression becomes higher as the value of the discriminant value becomes larger than the threshold value, whereas the severity of depression becomes lower as the value of the discriminant value becomes closer to the threshold value.

A person skilled in the art may appropriately decide the predetermined threshold value (cut-off value) according to various conditions such as a sex and age of the subject to be tested, the types of test sample, the types of biomarker, and the like. A method for determining a threshold value is not particularly limited, and for example, the threshold value can be determined according to a known technique.

The threshold value may be determined based only on results of measuring the biomarkers from a subject to be tested who is afflicted with depression (case subject to be tested), may be determined based only on results of measuring the biomarkers from a subject to be tested who is not afflicted with depression (control subject to be tested), or may be determined based on calculated discriminant values of the case subject to be tested and the control subject to be tested by measuring the biomarkers of both subjects. It is preferable that the threshold value be determined based on the calculated discriminant values of the case subject to be tested and the control subject to be tested by measuring the biomarkers of the biomarkers of both subjects. As long as the control subject to be tested is not afflicted with depression, the control subject to be tested may be afflicted with other diseases and may not be afflicted with other diseases but is preferably not afflicted with diseases correlated with the biomarker to be measured.

For example, when measuring the biomarkers of the control subject to be tested and determining a threshold value based only on the calculated discriminant value, the concentrations of the biomarkers measured in a plurality of individuals of the control subjects to be tested may be measured so as to determine a threshold value so that a range from an upper limit to a lower limit of the calculated discriminant value falls within a range of a normal value, or the concentrations of the markers measured in the plurality of individuals of the control subjects to be tested may be measured so as to determine a threshold value so that a range of an average value ± standard deviation of the calculated discriminant value falls within the range of the normal value.

Furthermore, for example, the concentrations of the biomarkers measured in the plurality of individuals of the control subjects to be tested may be measured so as to determine a threshold value such that the control subject to be tested is included within the range of the normal value at a predetermined ratio in the distribution of the calculated discriminant values. The predetermined ratio is, for example, 70% or more, preferably 80% or more, more preferably 90% or more, even more preferably 95% or more, and particularly preferably 100%. The above description can also be applied *mutatis mutandis* to cases where the biomarker of the case subjects to be tested is measured so as to determine a threshold value based only on the calculated discriminant value.

It is determined for each biomarker whether an abnormal value is set to a larger side or a smaller side compared with the normal value, and therefore the threshold value is set by taking the value into consideration.

When the biomarkers of the case subject to be tested and the control subject to be tested are measured so as to determine a threshold value based on the calculated discriminant values of both subjects, for example, a threshold value may be determined such that the control subject to be tested is included within the range of the normal value at a predetermined ratio and the case subject to be tested is included within the range of the abnormal value at a predetermined ratio. Specifically, for example, in a case of a biomarker having a high possibility of depression when the measurement value is equal to or higher than the threshold value, a threshold value can be determined such that the case subject to be tested is included at a predetermined ratio equal to higher than the threshold value and the control subject to be tested is included at a predetermined ratio below the threshold value.

More specifically, for example, in the case of a biomarker having a high possibility of depression when the measurement value is equal to or less than the threshold value, a threshold value can be determined such that the case subject to be tested is included at a predetermined ratio equal to lower than the threshold value and the control subject to be tested is included at a predetermined ratio above the threshold value.

Both the ratio of the case subjects to be tested showing the abnormal value and the ratio of the control subjects to be tested showing the normal value are preferably high. These ratios are, for example, 70% or more, preferably 80% or more, more preferably 90% or more, even more preferably 95% or more, and may be 100%. The higher these ratios become, the higher the specificity and sensitivity become.

Both specificity and sensitivity are preferably high. The specificity and the sensitivity are, for example, 70% or more, preferably 80% or more, more preferably 90% or more, even more preferably 95% or more, and particularly preferably 100%.

In the present specification, the term "specificity" means a rate that becomes negative in the control subject to be tested, and the higher the specificity becomes, the lower a false-positive rate becomes.

In addition, the term "sensitivity" means a rate that becomes positive in the case subject to be tested, and the higher the sensitivity becomes, the lower a false-negative rate becomes.

When both the specificity and the sensitivity cannot be increased, a threshold value may be set so that any one of the specificity and the sensitivity becomes high according to the purpose of testing depression, or the like. For example, in a case of aiming for establishing depression when a test result is positive, a threshold may be set so that the specificity becomes high. Furthermore, for example, in a case of aiming for excluding depression when a test result is negative, a threshold value may be set so that the sensitivity becomes high.

The threshold value may be determined using commercially available software. For example, using statistical analysis software, the threshold value that allows statistically the most appropriate discrimination between the control subject to be tested and the case subject to be tested may be determined.

In addition, in the evaluation step, evaluation of the severity of depression using the discriminant values calculated from the blood concentrations of the above-described biomarkers in the subject to be tested may be combined with other tests of depression so as to evaluate depression. Examples of the other tests of depression include a test of depression by interview by questionnaire (for example, Hamilton Rating Scale for Depression (HAMD) and the like) and a self-administered questionnaire (for example, Patient Health Questionnaire (PHQ)- 9, Beck Depression Inventory-II (BDI-2)), a test of depression using genes, proteins, and compounds, which are correlated with depression, as indicators, and the like.

<Program for Evaluating Severity of Depression>

In one the embodiment, the present invention provides a program for evaluating the severity of depression, which causes a computer to execute: an acquisition step of acquiring concentrations of the above-described biomarkers in blood collected from a subject to be tested; a discriminant value calculation step of calculating a discriminant value which is a value of a multivariate discriminant based on a blood concentration of at least one biomarker of the blood concentrations of the biomarkers acquired in the acquisition step and the multivariate discriminant set in advance which has the blood concentration of the biomarker as a variable and has at least one biomarker as the variable; an evaluation step of evaluating the severity of depression of the subject to be tested based on the discriminant value calculated in the discriminant value calculation step; and an output step of outputting the obtained evaluation results.

According to the evaluation program of the present embodiment, it is possible to easily and accurately evaluate the severity of depression automatically.

In the present specification, the term "program" means a data-processing method described in an arbitrary language and description method, in which the form such as a source code and a binary code is not limited.

The term "program" is not necessarily limited to a program configured as a single program, and includes a program having a distributed configuration as a plurality of modules or libraries, and a program in cooperation with a separate program represented by an OS (Operating System) so as to achieve functions of the program. The program is recorded on a recording medium and mechanically read by a computer or the like as necessary. Well-known configurations and procedures can be used for a specific configuration for reading the program recorded on the recording medium by each apparatus, a reading procedure, an installation procedure after reading, and the like.

In addition, in the present specification, the term "recording medium" includes an arbitrary "portable physical medium", an arbitrary "fixed physical medium", or a "communication medium."

Examples of the "portable physical medium" include, but are not limited to, a floppy (registered trademark) disk, a magneto-optical disk, a CD-ROM, a CD-R/W, a DVD-ROM, a DVD-R/W, a DVD-RAM, a DAT, an 8 mm tape, a memory card, a hard disk, a read-only memory (ROM), an SSD, a USB memory, and the like. Examples of the "fixed physical medium" include ROM, RAM, HD, and the like which are built in various computer systems. Examples of the "communication medium" include a device that holds a program for a short period of time such as a communication line and a carrier wave in a case of transmitting a program via a network such as LAN, WAN, and Internet, and the like.

[Acquisition Step] First, a measurement value of concentrations of the above-described biomarkers in blood collected from the subject to be tested is input from the outside so as to be acquired.

In the acquisition step of the present embodiment, acquired measurement data of the blood concentrations of the biomarkers relate to a concentration value of the biomarkers which is obtained by analyzing blood collected from the subject to be tested in advance.

A method for analyzing the biomarkers in blood will be simply described.

First, a blood sample is collected in a heparin-treated tube, and then the tube is subjected to centrifugation so as to separate the plasma.

All separated plasma samples may be stored frozen at -70°C until measurement of the concentrations of the biomarkers.

At the time of measuring the concentrations of the biomarkers, sulfosalicylic acid (final concentration: about 3%) is added to the plasma samples so as to perform deproteinization treatment.

For measurement of the concentrations of the biomarkers, an analytical instrument may be used according to the types of biomarker. Specifically, for example, an NMR apparatus, various mass spectrometers (GC-MS, LC-MS, CE-TOFMS, and the like), capillary electrophoresis, and the like may be used alone or in appropriate combination.

[Discriminant Value Calculation Step] Next, a discriminant value which is a value of a multivariate discriminant is calculated based on a blood concentration of at least one biomarker of the acquired blood concentrations of the biomarkers and the multivariate discriminant set in advance which has the blood concentration of the biomarker as a variable and has at least one biomarker as the variable.

The types of multivariate discriminant, the types of biomarker used for calculating the discriminant value, and the like are as described in the above section <Method for Evaluating Severity of Depression>.

The outline of a method for creating a multivariate discriminant (Step 1 to Step 4) will be described in detail.

(Step 1)

First, a candidate multivariate discriminant which is a candidate of a multivariate discriminant (for example, a linear discriminant expressed by the Equation (1), and the like) is created from the acquired blood concentrations of the biomarkers and, if necessary, biological information of the subject to be tested, based on a predetermined method for creating an equation.

In step 1, a plurality of candidate multivariate discriminants may be created from the acquired blood concentrations of the biomarkers and, if necessary, biological information of the subject to be tested using a plurality of different methods for creating an equation (including methods relating to multiple regression analysis such as principal component analysis, discriminant analysis, support vector machine, logistic regression analysis, k-means method, cluster analysis, and decision tree) in combination.

Specifically, with respect to the blood concentrations of the biomarkers obtained by analyzing blood obtained from a plurality of groups of healthy subjects and groups of patients with depression, and, if necessary, depression status information which is multivariate data composed of biological information of the subjects to be tested, a plurality of groups of the candidate multivariate discriminants may be concurrently created by using a plurality of different algorithms. For example, discriminant analysis and logistic regression analysis may be simultaneously performed using different algorithms so as to create two different candidate multivariate discriminants. In addition, the candidate multivariate discriminants may be created by converting the depression status information by using the candidate multivariate discriminants created by performing principal component analysis and performing discriminant analysis on the converted depression status information.

In this manner, it is possible to finally create an appropriate multivariate discriminant that is suitable for diagnostic conditions.

The candidate multivariate discriminant created by using principal component analysis is a linear equation made up of each biomarker variable that maximizes variance of the blood concentration data of all the biomarkers.

In addition, the candidate multivariate discriminant created using the discriminant analysis is a higher-order equation (including an index and a logarithm) made up of each biomarker variable that minimizes a ratio of a sum of variances within each group to the variance of the blood concentration data of all the biomarkers.

Furthermore, the candidate multivariate discriminant created using the support vector machine is a higher-order equation (including a kernel function) made up of each biomarker variable that maximizes the boundary between groups.

Furthermore, the candidate multivariate discriminant created using multiple regression analysis is a higher-order equation made up of each biomarker variable that minimizes a sum of distances from the blood concentration data of all the biomarkers.

Furthermore, the candidate multivariate discriminant created by using logistic regression analysis is a fractional expression having, as a term, a natural logarithm whose index is a linear equation made up each biomarker variable that maximizes likelihood.

Furthermore, the k-means method is a method for searching k neighborhood of each biomarker concentration data, defining the most occurring group among groups to which neighboring points belong as a group to which the data thereof belongs, and therefore selecting biomarker variables by which the group to which the input blood concentration data of the biomarkers belong, and the defined groups become most coincident with each other.

Furthermore, the cluster analysis is a method for clustering (grouping) points which are at the nearest distance with each other in the blood concentration data of all the biomarkers.

Furthermore, the decision tree is a method for predicting a group of the blood concentration data of the biomarkers from a pattern in which biomarker variables are ranked so as to use a biomarker variable having a higher ranking.

(Step 2)

Next, the candidate multivariate discriminant created in step 1 is verified (mutually verified) based on a predetermined verification method. The verification of the candidate multivariate discriminant is performed on each candidate multivariate discriminant created in step 1.

In step 2, for example, at least one of a discrimination rate, sensitivity, and specificity of the candidate multivariate discriminant, and an information criterion may be verified based on, for example, at least one of the group consisting of a bootstrapping method, a holdout method, and a leave-one-out method. Accordingly, it is possible to create a candidate multivariate discriminant having a high level of predictability or robustness, in which depression status information and diagnostic conditions are taken into consideration.

In the present specification, the term "discrimination rate" means that a correct rate of the depression status evaluated using the evaluation method of the present embodiment among all input data.

In addition, the term "sensitivity of the multivariate discriminant" means that a correct rate (that is, a rate in which patients with depression are evaluated as being afflicted with depression) of the depression status evaluated using the evaluation method of the present embodiment among patients diagnosed with depression, which are described in the input data (that is, data of patients with depression).

Furthermore, the term "specificity of the multivariate discriminant" means that a correct rate (that is, a rate in which healthy subjects are evaluated as being normal) of the depression status evaluated using the evaluation method of the present embodiment among patients diagnosed as not having depression, which are described in the input data (that is, data of healthy subjects).

In addition, the term "information criterion" means that the number of biomarker variables of the candidate multivariate discriminants created in step 1 is added with a difference between the depression status evaluated in the present embodiment and the depression status described in the input data.

Furthermore, the term "predictability" indicates an average of the discrimination rate, the sensitivity, and the specificity, which is obtained by repeating the verification of the candidate multivariate discriminant.

Furthermore, the term "robustness" indicates a variance of the discrimination rate, the sensitivity, and the specificity, which is obtained by repeating the verification of the candidate multivariate discriminant.

(Step 3)

Next, by selecting the variable of the candidate multivariate discriminant from the verification result in step 2 based on a predetermined method for selecting a variable, a combination of the blood concentration data of the biomarkers, which is contained in the depression status information used for creating the candidate multivariate discriminant, is selected. The selection of the biomarker variables is performed on each candidate multivariate discriminant created in step 1. Accordingly, it is possible to appropriately select the biomarker variable of the candidate multivariate discriminant. Subsequently, step 1 is performed again using the depression status information including the blood concentration data of the biomarkers selected in step 3.

In addition, in step 3, the biomarker variable of the candidate multivariate discriminant may be selected from the verification result in step 2 based on at least one of a stepwise method, a best path method, a neighborhood search method, and a genetic algorithm.

In the present specification, the term "best path method" means a method in which amino acid variables included in the candidate multivariate discriminant are sequentially reduced one by one, and evaluation indices given by the candidate multivariate discriminant are optimized so as to select an amino acid variable.

(Step 4)

Step 1, step 2, and step 3 described above are repeatedly performed, and based on the accumulated verification results, the candidate multivariate discriminant to be adopted as the multivariate discriminants is selected from the plurality of candidate multivariate discriminants, and therefore a multivariate discriminant is created.

Examples of the selection of the candidate multivariate discriminant include a case in which an optimal candidate multivariate discriminant is selected from the candidate multivariate discriminants created by the same method for creating an equation, and a case in which an optimal candidate multivariate discriminant is selected from all candidate multivariate discriminants.

As described above, in the method for creating a multivariate discriminant, based on the depression status information, the process related to the creation of the candidate multivariate discriminant, the verification of the candidate multivariate discriminant, and the selection of the variable of the candidate multivariate discriminant is systematized in a series of flows so as to be performed, and therefore it is possible to create a multivariate discriminant which is the most optimized for the evaluation of the severity of depression. In other words, in the process of creating the multivariate discriminant, the blood concentrations of the biomarkers are used for multivariate statistical analysis, a variable selection method and cross-validation are combined in order to select an optimum and robust variable pair, and therefore a multivariate discriminant having a high level of diagnostic performance is extracted.

As the multivariate discriminant, for example, logistic regression, linear discriminant, support vector machine, the Mahalanobis distance method, multiple regression analysis, cluster analysis, and the like can be used.

[Evaluation Step] Subsequently, the severity of depression of the subject to be tested is evaluated based on the calculated discriminant value.

Specifically, by comparing the discriminant value with a predetermined threshold value (cut-off value), it is possible to discriminate whether a patient is in a group of patients with depression or a group of patients not having depression (healthy subject group).

Furthermore, it is possible to evaluate that the severity of depression becomes higher as the value of the discriminant value becomes larger than the threshold value, whereas the severity of depression becomes lower as the value of the discriminant value becomes closer to the threshold value.

The predetermined threshold value (cut-off value) is as described in the section [Evaluation Step] of the section <Method for Evaluating Severity of Depression>.

[Output Step] Next, the obtained evaluation results are output.

For example, on a computer, when the process from the acquisition step to the evaluation step is performed, the evaluation results are output on a monitor screen of the computer. Accordingly, medical personnel can acquire information on the evaluation results.

In addition, the evaluation results output on the monitor screen of the computer may be printed by a printer or the like.

[Measurement Step] A step of causing a biomarker-measuring apparatus to measure the concentrations of the biomarkers in blood may be further included before the acquisition step.

As the biomarker-measuring apparatus, measuring equipment may be used according to the types of biomarker. Specifically, for example, an NMR apparatus, various mass spectrometers (GC-MS, LC-MS, CE-TOFMS, and the like), capillary electrophoresis, and the like may be used alone or in appropriate combination.

The medical personnel collect a blood sample from the subject to be tested, preprocesses the sample if necessary, and set the blood sample in the biomarker-measuring apparatus, and therefore the program of the present embodiment automatically is allowed to measure the concentrations of the biomarkers in the blood sample. The measured blood concentrations of the biomarkers are used in the subsequent acquisition step.

In addition, a computer that executes the program of the present embodiment may be adopted as a system for evaluating the severity of depression.

<Biomarker for Predicting Various symptoms of Depression>

In the embodiment, the present invention provides a biomarker for predicting various symptoms of the subject to be tested in at least any one of Patient Health Questionnaire (PHQ)-9, Beck Depression Inventory-II (BDI-2), and the Hamilton Rating Scale for Depression (HAMD), which are used for the diagnosis of depression at the present.

According to the biomarkers of the present embodiment, various symptoms of depression of the subject to be tested in at least one of PHQ-9, BDI-2, and HAMD can be easily and accurately predicted.

Combinations of various symptoms and a specific biomarker for predicting the symptoms are as shown below.

(1) Biomarker for Predicting Loss of Interest/Pleasure

At least one compound selected from the group consisting of acetylcarnitine, urocanic acid, 2-oxobutanoate, carbamoyl phosphate, proline, and 3-methylhistidine.

(2) Biomarker for Predicting Depressive Feelings

At least one compound selected from the group consisting of N-acetylglutamate, 2-oxobutanoate, carnosine, 5-hydroxytryptophan, proline, and melatonin.

(3) Biomarker for Predicting Feelings of Worthlessness/Guilt

At least one compound selected from the group consisting of agmatine, ATP, argininosuccinate, tryptophan, valine, 5-hydroxytryptophan, proline, and phosphoenolpyruvate.

(4) Biomarker for Predicting Agitation/Retardation (Unstable Mood)

At least one compound selected from the group consisting of citrate, creatine, 5-hydroxytryptophan, 4-hydroxyproline, 3-hydroxybutyrate, fumarate, proline, and leucine.

(5) Biomarker for Predicting Suicide Attempts

At least one compound selected from the group consisting of N-acetylglutamate, alanine, xanthurenate, xanthosine, kynurenine, kynurenate, citrate, 3-hydroxykynurenine, phenylalanine, and phosphoenolpyruvate.

(6) Biomarker for Predicting Sleep Disorder

At least one compound selected from the group consisting of agmatine, N-acetylaspartate, N-acetylglutamine, adenine, AMP, ATP, isocitrate, ornithine, carnitine, citrate, glucosamine, $\beta$-glycerophosphate, serotonin, tyrosine, threonine, pyruvate, pyroglutamate, phenylalanine, fumarate, pantothenate, 2-phosphoglycerate, PRPP, methionine, and 3-methylhistidine.

(7) Biomarker for Predicting Fatigue

At least one compound selected from the group consisting of asparagine, N-acetylaspartate, 2-oxobutyrate, ornithine, $\beta$-glycerophosphate, melatonin, and proline.

<Method for Predicting Various Symptoms of Depression>

In the embodiment, the present invention provides a method for predicting various symptoms of depression of the subject to be tested in at least one of PHQ-9, BDI-2, and HAMD, which are used for the diagnosis of depression at the present, in which the blood concentrations of the biomarkers for predicting various symptoms of depression as described above are used.

According to the prediction method of the present embodiment, various symptoms of depression of the subject to be tested in at least one of PHQ-9, BDI-2, and HAMD can be easily and accurately predicted.

Specifically, the prediction method of the present embodiment is as follows.

First, a blood sample is collected from the subject to be tested and the blood concentrations of the biomarkers for predicting various symptoms of depression as described above are measured. The measurement method is the same as that of the section [Measurement Step] described in the section <Method for Evaluating Severity of Depression>.

The biomarkers to be measured can be selected appropriately according to the types of symptoms of depression to be predicted.

For example, if the symptom to be predicted is loss of interest/pleasure, the biomarker to be measured is at least one compound selected from the group consisting of acetylcarnitine, urocanic acid, 2-oxobutanoate, carbamoyl phosphate, proline, and 3-methylhistidine (hereinafter, will be referred to as "biomarker group for predicting loss of interest/pleasure" in some cases). It is preferable to measure two or more biomarkers among the biomarker group for predicting loss of interest/pleasure, and it is more preferable to measure all the biomarkers among the biomarker group for predicting loss of interest/pleasure.

By measuring the plurality of biomarkers in combination, it is possible to improve the prediction accuracy of loss of interest/pleasure in the subject to be tested.

Next, using the measured blood concentrations of the biomarkers for predicting the various symptoms of depression, in the same manner as the section [Discriminant Value Calculation Step] described in the section <Method for Evaluating Severity of Depression>, a discriminant value which is a value of a multivariate discriminant is calculated based on the multivariate discriminant set in advance which has at least one biomarker as the variable.

In addition, the biomarkers used for the multivariate discriminant may be selected appropriately according to the types of symptoms of depression to be predicted.

For example, if the symptom to be predicted is loss of interest/pleasure, the biomarker to be measured is at least one compound selected from the group consisting of acetylcarnitine, urocanic acid, 2-oxobutanoate, carbamoyl phosphate, proline, and 3-methylhistidine (hereinafter, will be referred to as "biomarker group for predicting loss of interest/pleasure" in some cases). It is preferable to measure two or more biomarkers among the biomarker group for predicting loss of interest/pleasure, and it is more preferable to measure all the biomarkers among the biomarker group for predicting loss of interest/pleasure.

By incorporating the plurality of biomarkers into the multivariate discriminant, it is possible to improve the prediction accuracy of loss of interest/pleasure in the subject to be tested.

In addition, as a variable in the multivariate discriminant, biological information of other subjects to be tested (for example, biological metabolites such as minerals and hormones, gender, age, eating habits, drinking habits, fitness habits, degree of obesity, history of disease, interview data, and the like) may be further used, in addition to the biomarkers.

Subsequently, the presence or absence of a specific symptom of depression of the subject to be tested is predicted based on the calculated discriminant value.

Specifically, by comparing the discriminant value with a predetermined threshold value (cut-off value), it is possible to discriminate whether the group is the group having the specific symptom of depression or the group not having the specific symptom of depression.

Furthermore, it is possible to predict that the specific symptom of depression is exhibited stronger as the value of the discriminant value becomes larger than the threshold value, whereas the specific symptom of depression is exhibited weaker as the value of the discriminant value becomes closer to the threshold value.

The predetermined threshold value (cut-off value) can be obtained by using the same method as that of the section [Evaluation Step] of the section <Method for Evaluating Severity of Depression>.

In addition, the predictions of various symptoms of depression using the discriminant values calculated from the blood concentrations of the biomarkers for predicting various symptoms of depression in the subjects to be tested, and other tests of depression may be combined so as to predict the presence or absence of various symptoms of depression.

Examples of the other tests of depression include a test of depression by interview by questionnaire (for example, Hamilton Rating Scale for Depression (HAMD) and the like) and a self-administered questionnaire (for example, Patient Health Questionnaire (PHQ)- 9, Beck Depression Inventory-II (BDI-2)), a test of depression using genes, proteins, and compounds, which are correlated with depression, as indicators, and the like.

In addition, it is possible to create a program for predicting various symptoms of depression by using the same method as that of the section <Program for Evaluating Severity of Depression>, except that the biomarkers for predicting various symptoms of depression are used instead of the biomarkers for evaluating the severity of depression.

For example, in a case of using the biomarkers for predicting suicidal ideation (suicide attempts), it is possible to predict whether the group is the group having suicide attempts or the group not having suicide attempts by comparing the discriminant value with a predetermined threshold value (cut-off value).

Furthermore, it is possible to predict that suicide attempts from depression are exhibited stronger as the value of the discriminant value becomes larger than the threshold value, whereas suicide attempts from depression are exhibited weaker as the value of the discriminant value becomes closer to the threshold value.

<Usage of Biomarkers for Evaluating Severity of Depression and Biomarkers for Predicting Various Symptoms of Depression>

In the present embodiment, the biomarkers described above can be used for the following applications, in addition to evaluating the severity of depression and predicting various symptoms of depression.

[Determination of Efficacy of Drug] In one embodiment, the present invention provides a method for determining efficacy of a therapeutic agent for depression, in which the biomarkers for evaluating the severity of depression and the biomarkers for predicting various symptoms of depression are used.

Generally, the effect of drugs for disease may vary depending on individuals.

Therefore, research on the efficacy of a certain therapeutic agent for each individual is significantly useful, and by using the above-described biomarkers, it is possible to easily research the efficacy of a therapeutic agent.

For example, before and after medicating with a therapeutic agent for depression, blood is collected from a patient with depression so as to measure contents of the above-described biomarkers contained in the collected blood, and therefore the contents of the above-described biomarkers in the blood before and after medicating with the therapeutic agent are compared. If the contents of the above-described biomarkers approach a normal range after medicating with the therapeutic agent, it is possible to determine that the therapeutic agent is effective.

As described above, by using the above-described biomarkers, it is possible to easily determine whether or not the therapeutic agent is effective.

Examples

Hereinafter, the present invention will be described in detail based on examples, comparative examples, and the like, but the present invention is not limited to the examples and the like.

[Example 1] [1] Selection of Patients with Depression (Diagnosis of Depression) and Collection of Plasma Sample

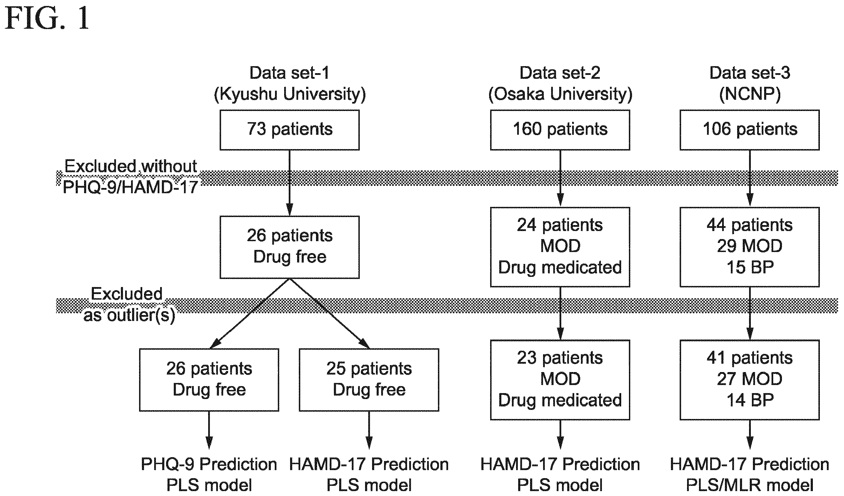

In three institutes of Kyushu University, Osaka University, and National Center of Neurology and Psychiatry, data of patients with depression in the present example were used after written informed consent was obtained from all patients with depression. A method for selecting patients in each institute is as follows. FIG. 1 shows a selection flow of plasma samples of the patients with depression in each institute.

(1) Data Set-1 (Kyushu University)

In addition, among 73 patients of Kyushu University, plasma was collected from 26 unmedicated new psychiatric patients with depression and used for production of a regression model capable of predicting the severity of depression in PHQ-9. In addition, plasma was collected from 25 patients among the 26 patients and used for production of a regression model capable of predicting the severity of depression in HAMD-17.
Each patient was diagnosed with depression by using the Structured Clinical Interview for Diagnosis (SCID) interview according to DSM-IV criteria performed by a trained psychiatrist.

(2) Data Set-2 (Osaka University)

In addition, among 160 patients of Osaka University, plasma was collected from 23 medicated patients diagnosed with major depressive disorders (MDD) (that is, "depression") and used for production of a regression model capable of predicting the severity of depression in HAMD-17.
Each patient was diagnosed with depression by using the SCID interview performed by two trained psychiatrists.

(3) Data Set-3 (National Center of Neurology and Psychiatry)

In addition, among 106 patients of National Center of Neurology and Psychiatry, plasma was collected from medicated and unmedicated patients (41 patients) diagnosed with MDD (27 patients) or bipolar disorder (14 patients) and used for production of a regression model capable of predicting the severity of depression in HAMD-17.
A structured interview was conducted with each patient using the Mini-International Neuropsychiatric Interview (M.I.N.I.) performed by a trained psychologist or psychiatrist. In addition, diagnosis of MDD or bipolar disorder was determined based on M.I.N.I. interviews, additional unstructured interviews, and information in medical records according to DSM-IV criteria.

(4) Collection of Plasma Sample

Furthermore, the collection of the plasma samples was performed by peripheral blood sampling via venipuncture.

[2] Diagnosis of Severity of Depression

Subsequently, while collecting the plasma samples, the severity of depression was evaluated by using Japanese version of HAMD-17 (all institutes) and PHQ-9 (only Kyushu University).
All scores of HAMD-17 were evaluated by a trained psychiatrist or clinical psychotherapist. In addition, the PHQ-9 questionnaire was filled out by the patients themselves so as to be evaluated.

[3] Metabolomic Analysis

Subsequently, the analysis was performed with the plasma samples by liquid chromatography mass spectrometry (LC-MS). Specifically, plasma metabolites were analyzed using the triple quad LCMS-8040 (manufactured by Shimadzu Corporation) in reversed-phase ion chromatography and hydrophilic interaction chromatography modes.
For the reversed-phase ion chromatography, an ACQUITY UPLC BEH C18 column (100 Å to 2.1 mm, particle size of 1.7 $\mu$m, manufactured by Waters Corporation) was used.
A mobile phase consisting of solvent A (15 mM acetic acid, 10 mM tributylamine) and solvent B (methanol) was used. The column oven temperature was set at 40°C.
A gradient elution program was as follows. Flow rate 0.3 mL/min: 0 to 3 minutes, 0% solvent B: 3 to 5 minutes, 0% to 40% solvent B: 5 to 7 minutes, 40% to 100% solvent B: 7 to 10 minutes, 100% solvent B: 10.1 to 14 minutes, 0% solvent B.
In addition, parameters of negative ESI mode under multiple reaction monitoring were as follows. Drying gas flow rate 15 L/min; nebulizer gas flow rate 3 L/min; DL temperature 250°C; heat block temperature 400°C; collision energy (CE) 230 kPa.
Furthermore, for the hydrophilic interaction chromatography, the Luna 3u HILIC 200A column (150 Å to 2 mm, particle size of 3 $\mu$m, manufactured by Phenomenex Inc.) was used.

A mobile phase consisting of solvent A (10 mM ammonium formate solution) and solvent B (acetonitrile:10 mM ammonium formate solution = 9:1) was used. The column oven temperature was set at 40°C.

A gradient elution program was as follows. Flow rate 0.3 mL/min: 0 to 2.5 minutes, 100% solvent B: 2.5 to 4 minutes, 100% to 50% solvent B: 4 to 7.5 minutes, 50% to 5% solvent B: 7.5 to 10 minutes, 5% solvent B: 10.1 to 12.5 min, 100% solvent B.

In addition, parameters of positive and negative ESI modes under multiple reaction monitoring were the same as those of the reversed-phase ion chromatography.

[4] Production of Regression Model Capable of Processing Data of Metabolites and Predicting Severity of Depression

Subsequently, a metabolomic data process including peak detection and retention time was performed using the LabSolutions LC-MS software program (manufactured by Shimadzu Corporation).

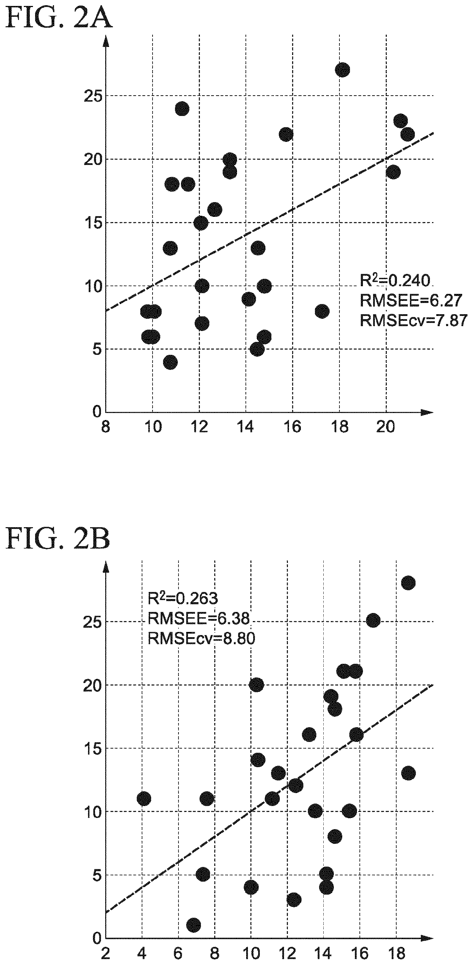

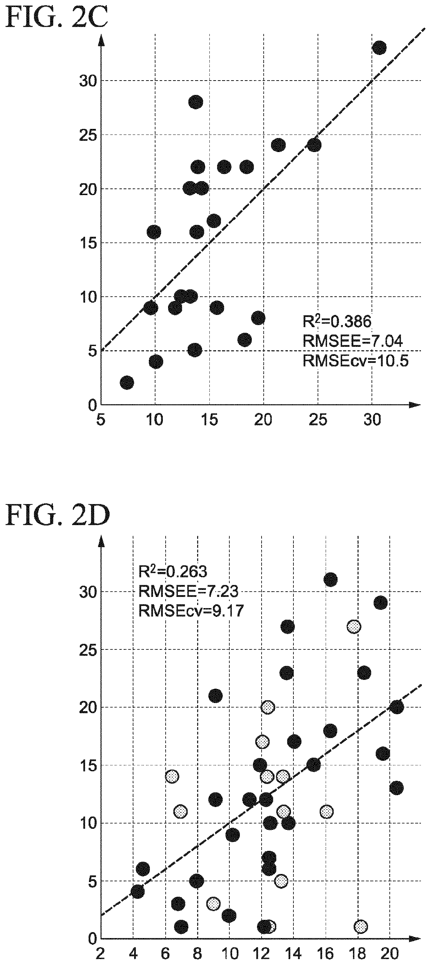

In addition, in multivariate data in each data set, after pretreatment with Pareto scaling, biomarkers related to the severity of depression were separated, and therefore a regression model capable of predicting the severity of depression was produced by using SIMCA 14.0 software (manufactured by Umetrics). FIG. 2A is a graph showing the relationship between measurement values of scores of PHQ-9 and predictive values obtained by a regression model for predicting scores of PHQ-9 in the data set-1, and FIG. 2B is a graph showing the relationship between measurement values of scores of HAMD-17 and predictive values obtained by a regression model for predicting scores of HAMD-17 in the data set-1. In addition, FIG. 2C is a graph showing the relationship between measurement values of scores of HAMD-17 and predictive values obtained by a regression model for predicting scores of HAMD-17 in the data set-2, and FIG. 2D is a graph showing the relationship between measurement values of scores of HAMD-17 and predictive values obtained by a regression model for predicting scores of HAMD-17 in the data set-3.

An X-axis of FIG. 2A represents the scores of PHQ-9 measured by responses of the self-administered questionnaire by patients, and a Y-axis represents the scores of PHQ-9 predicted from multivariate data of the metabolites. Furthermore, in FIGS. 2B, 2C, and 2D, X-axes represent the scores of HAMD-17 diagnosed by a psychiatrist or clinical psychotherapist, and Y-axes represent the scores of HAMD-17 predicted from multivariate data of the metabolites.

Furthermore, in FIG. 2D, black circles represent values in patients diagnosed with depression and gray circles represent values in patients diagnosed with bipolar disorder.

As results of LC-MS, 123 metabolites were detected from the plasma samples of the 26 unmedicated new psychiatric patients with depression of the data set-1.

In addition, based on FIGS. 2A and 2B, favorable correlation was observed between the measurement values and the predictive values in both PHQ-9 ($R^2$ = 0.24) and HAMD-17 ($R^2$ = 0.263).

Furthermore, based on FIG. 2C, a stronger correlation ($R^2$ = 0.386) was observed between the measurement values and the predictive values of HAMD-17 in the data set-2, compared to the correlation ($R^2$ = 0.263) between the measurement values and the predictive values of HAMD-17 in the data set-1.

Furthermore, based on FIG. 2D, the level of correlation was $R^2$ = 0.263 between the measurement values and the predictive values of HAMD-17 in the data set-3, which was the same level as the correlation ($R^2$=0.263 and $R^2$=0.386) between the measurement values and the predictive values of HAMD-17 in the data set-1 and the data set-2.

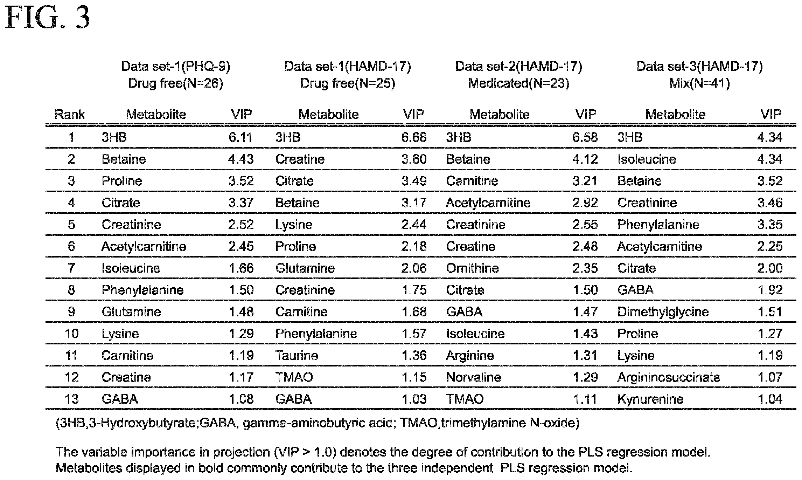

In addition, FIG. 3 shows metabolites having a high degree of contribution (variable importance in projection (VIP) > 1.0) to the predictive values obtained by a regression model for predicting the scores of PHQ-9 or HAMD-17 in the data sets-1 to 3.

In FIG. 3, 3HB represents 3-hydroxybutyrate, GABA represents 4-aminobutyric acid ($\gamma$ (gamma)-aminobutyric acid), and TMAO represents trimethyloxamine.

Based on FIG. 3, in the data set-2 of the medicated patients, 74% of the metabolites having a high degree of contribution to the predictive values overlapped those in the data set-1 of the unmedicated patients.

In addition, in the data set-3 of the medicated and unmedicated patients, the metabolites having a high degree of contribution to the predictive values were almost the same as those in the data set-1 and the data set-2.

Among these metabolites, 5 metabolites of 3-hydroxybutyrate, betaine, citrate, creatinine, and GABA were common in all the three data sets regardless of the medication condition and differences in treatment, and thus it became clear that 5 metabolites were related to the severity of depression.

In particular, 3-hydroxybutyrate is a metabolite having the highest degree of contribution to the predictive values in the three data sets, and thus it became clear that 3-hydroxybutyrate shows positive correlation with a total score of HAND-17.

Based on the above results, it was found that the biomarkers clarified by the metabolomic analysis are useful for evaluating the severity of depression.

[5] Correlation analysis of Various Symptoms of Depression and Metabolites

Subsequently, in order to clarify metabolites related to various symptoms of depression, correlation analysis was performed on subscales (various symptoms of depression) of PHQ-9 or HAMD-17 and the 123 metabolites by using the data set-1. As a correlation analysis method, the same method as that of the correlation analysis of the severity of depression and the metabolites was used, which is described in the section "[4] Production of Regression Model Capable of Processing Data of Metabolites and Predicting Severity of Depression."

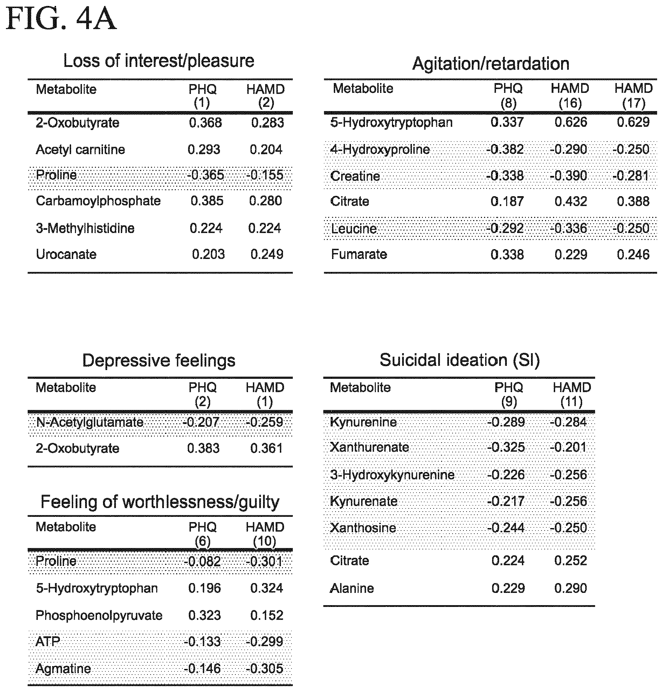

FIG. 4A shows, for each symptom, metabolites having a moderate correlation (an absolute value of a correlation coefficient is 0.3 or more) with various symptoms of depression.

In FIG. 4A, gray shaded metabolites represent that the metabolites have a negative correlation with the corresponding symptoms.

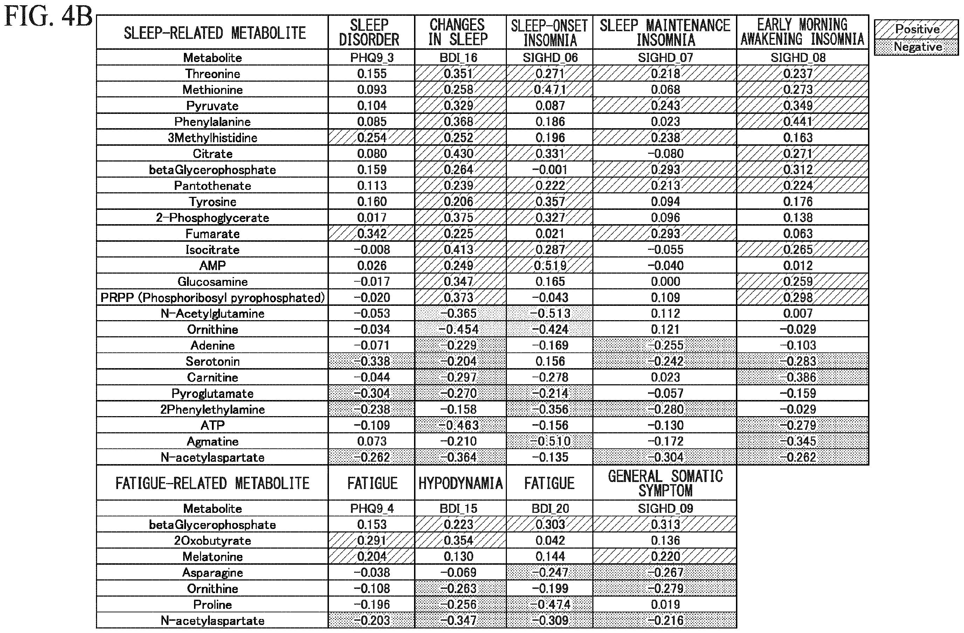

In addition, FIG. 4B shows metabolites having a moderate correlation (an absolute value of a correlation coefficient is 0.2 or more) with sleep disorders or fatigue in depression.

In FIG. 4B, values hatched with diagonal lines indicate that the metabolites have a positive correlation in which a correlation coefficient is 0.2 or more, and gray shaded values indicate a negative correlation in which a correlation coefficient is -0.2 or less.

Based on FIGS. 4A and 4B, it became clear that the types of metabolites were related to depression varied depends on the symptoms of depression.

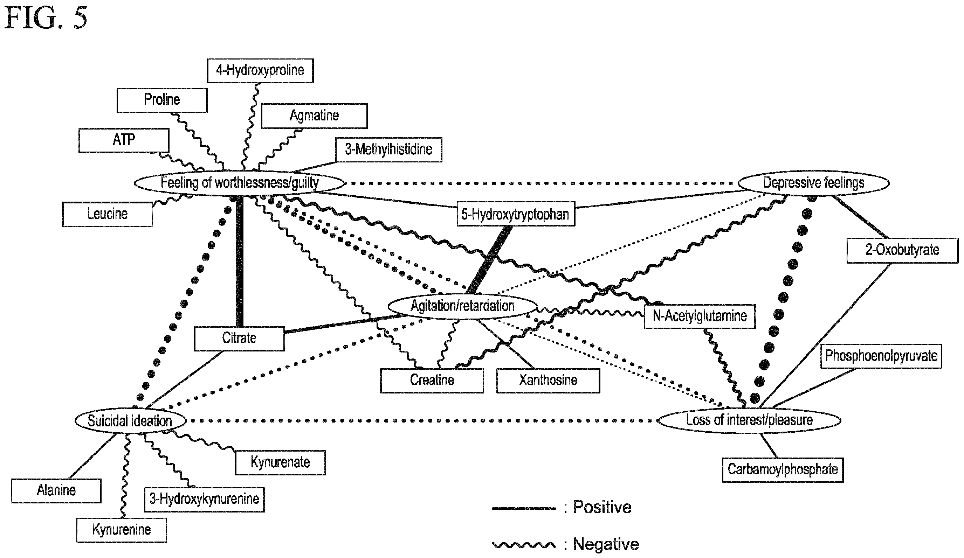

In addition, FIG. 5 shows a correlation network between the subscales (various symptoms of depression) of HAMD-17 in the data set-1 and the metabolites.

In FIG. 5, solid lines show a correlation between each metabolite and various symptoms of depression, and dotted lines show a correlation between various symptoms of depression. Furthermore, a thickness of the lines reflects the strength of the correlation, straight solid lines show a positive correlation, and wavy solid lines show a negative correlation.

Based on FIG. 5, for example, 2-oxobutyrate (one of hydroxy carboxylates containing 3-hydroxybutyrate) was related to "loss of interest" and "depressive feelings", whereas N-acetylglutamate was only related to "depressive feelings."

In addition, proline, 5-hydroxytryptophan, phosphoenolpyruvate, ATP, and agmatine were related to "feelings of worthlessness/guilt", and 5-hydroxytryptophan was also strongly related to "agitation/retardation (unstable mood)."

In addition, metabolites of the kynurenine pathway had a negative correlation with "suicidal ideation (SI) (suicide attempts)", whereas citrate and alanine had a positive correlation with SI.

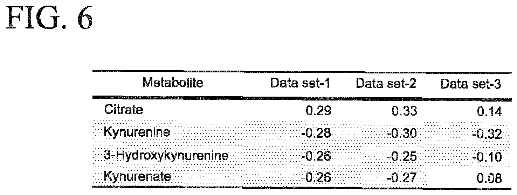

Next, with respect to the data set-2 and the data set-3, the correlation analysis between SI of HAMD-17 and metabolites was carried out in the same manner. FIG. 6 shows metabolites having a moderate correlation with SI of HAMD-17 in all the data sets-1 to 3. In FIG. 6, gray shaded metabolites represent the metabolites having a negative correlation with SI.

Based on FIG. 6 it became clear that in SI of HAMD-17 in data sets-2 and 3, citrate had a positive correlation, whereas the metabolites of the kynurenine pathway (especially kynurenine and 3- hydroxykynurenine) had a negative correlation, as well as data set-1.

[6] Production of Algorithm for Predicting Presence or Absence of SI

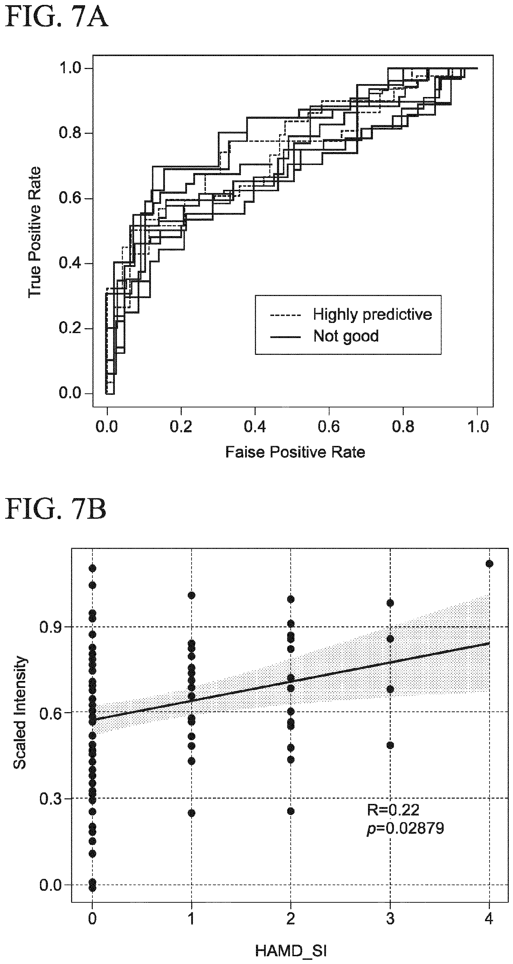

In depression, the presence or absence of SI is very important for preventing suicide. Therefore, analysis was further performed while focusing on SI and an algorithm for predicting the presence or absence of SI in patients with depression was produced. The algorithm for predicting the presence or absence of SI was produced using a machine-learning model. Specifically, ten types of training data among a total of 104 data (data including the data set-1, the data set-2, and the data set-3) were used for producing a prediction model by logistic regression, a support vector machine, or a random forest method (refer to FIG. 7A). In FIG. 7A, an X-axis represents "False Positive Rate" and a Y-axis represents "True Positive Rate." In addition, in FIG. 7A, the term "Highly predictive" (dotted line) represents a curve (true positive rate and true negative rate (true rate) > 0.7) having a high degree of contribution in order to produce a prediction model of the test data sets.

In addition, the "Fitting Ability" (degree of fit) was visualized by a ROC curve and evaluated by area under the curve (AUC). The "Prediction Ability" (degree of prediction) was evaluated based on the true positive rate and the true negative rate (true rate) in the test data sets.

The machine-learning model and statistical graphics were generated using R packages including ggplot2, e1071, randomForest, and ROC.

FIG. 7B is a graph showing a significant correlation (R = 0.22, p = 0.028) between suicide attempts of HAMD-17, and predictive values obtained by a regression model using a multiple linear discriminant having variables of citrate and kynurenine in plasma, of which the intensity has been standardized. In FIG. 7B, a linear regression line was drawn in an area of 95% degree of confidence (gray shaded part).

Based on FIG. 7B, three models (two logistic regression and one support vector machine) highly contributed to the prediction (AUC > 0.7, and true rate > 0.7).

In addition, developing the algorithm for predicting a degree of SI by using two metabolites of citrate and kynurenine as variables (R = 0.22, p = 0.028) was successful.

The detailed data are not shown, but it was possible to discriminate the presence or absence of SI in patients with depression at a prediction rate of 79% by using the algorithm.

[Example 2] [1] Selection of Patients with Depression (Diagnosis of Depression) and Collection of Plasma Sample

In Kyushu University, data of patients with depression in the present example were used after written informed consent was obtained from all patients with depression.

Plasma was collected from 22 unmedicated new psychiatric patients with depression (11 males, 11 females, average age of 31.18 years old (SD = 7.29)) and used for production of a regression model capable of predicting the severity of depression in Beck Depression Inventory (BDI-II). Each patient was diagnosed with depression by structured clinical interview SCID-I.

Furthermore, the collection of the plasma samples was performed by peripheral blood sampling via venipuncture.

[2] Diagnosis of Severity of Depression

Subsequently, while collecting the plasma samples, the severity of depression was evaluated by using Beck Depression Inventory (BDI-II).

[3] Metabolomic Analysis

Next, using the same method as in [3] of Example 1, the concentrations of the metabolites of the tryptophan and kynurenine pathway (indole carboxaldehyde, potassium indoleacetate, kynurenate, kynurenine, serotonin, tryptophan, xanthurenate), cholesterol (LDL-C, HDL-C), urea, total bilirubin, and cytokines (IL-1$\beta$, TNF-$\alpha$, IL-4, IL-6, IL-10, and IL-12) in plasma of each patient were measured.

[4] Production of Regression Model Capable of Processing Data of Metabolites and Predicting Severity of Depression

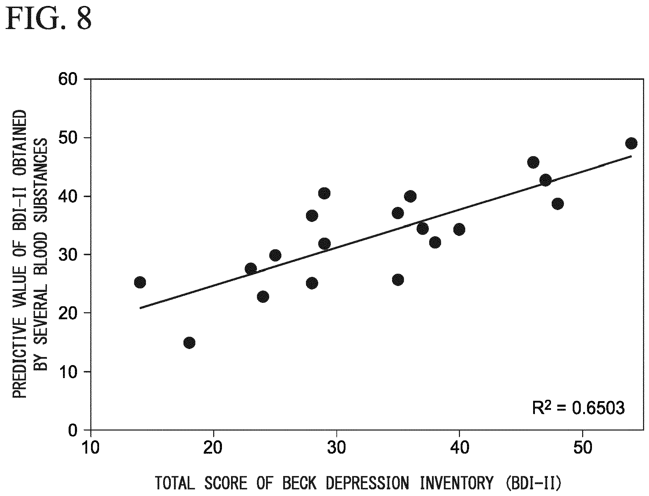

Next, using the same method as in [4] of Example 1, a regression model capable of predicting the severity of depression after data processing was produced. FIG. 8 is a graph showing the relationship between measurement values of scores of BDI-II and predictive values obtained by a regression model for predicting scores of BDI-II.

Based on FIG. 8, the level of correlation between the measurement value of BDI-II and the predictive value obtained by the regression model for predicting the scores of BDI-II was $R^2 = 0.6503$, which was a high level.

Based on the above results, it was found that an algorithm for predicting the severity of depression which has higher accuracy can be developed by adding the values of cholesterol, urea, cytokine, and the like as variables in addition to the metabolites obtained by the metabolomic analysis.

Industrial Applicability

The biomarkers of the present invention are objective biomarkers which are clinically useful, and therefore it is possible to simply evaluate the severity of depression. In addition, according to the biomarkers of the present invention, it is possible to evaluate various symptoms of depression such as depressive feelings, loss of interest, suicidal ideation, and feelings of guilt. Furthermore, based on the biomarkers of the present invention, the present invention can be applied to elucidation of the pathophysiological mechanism of depression.

Embodiments

1. A biomarker for evaluating the severity of depression, comprising:
at least one compound selected from the group consisting of 4-aminobutyric acid ($\gamma$ (gamma)-aminobutyric acid: GABA), arginine, argininosuccinate, isoleucine, indole carboxaldehyde, potassium indoleacetate, carnitine, acetylcarnitine, ornithine, xanthurenate, kynurenate, kynurenine, citrate, creatine, creatinine, glutamine, dimethylglycine, serotonin, taurine, trimethyloxamine (TMAO), tryptophan, norvaline, 3-hydroxybutyrate, phenylalanine, proline, betaine, and lysine.

2. The biomarker according to Embodiment 1, further comprising:
at least one compound selected from the group consisting of cholesterol, uric acid, bilirubin, and cytokines.

3. A method for evaluating the severity of depression, comprising:

a measurement step of measuring blood concentrations of biomarkers according to Embodiments 1 or 2 of a subject to be tested;
a discriminant value calculation step of calculating a discriminant value which is a value of a multivariate discriminant based on a blood concentration of at least one biomarker of the blood concentrations of the biomarkers measured in the measurement step and the multivariate discriminant set in advance which has the blood concentration of the biomarker as a variable and has at least one biomarker as the variable; and
an evaluation step of evaluating severity of depression of the subject to be tested based on the discriminant value calculated in the discriminant value calculation step.

4. The method for evaluating severity of depression according to Embodiment 3, wherein the multivariate discriminant is one fractional expression, a sum of a plurality of the fractional expressions, a logistic regression equation, a linear discriminant, a multiple regression equation, an equation created with a support vector machine, an equation created by the Mahalanobis distance method, an equation created by canonical discriminant analysis, or an equation created with a decision tree.

5. A program for evaluating severity of depression, which causes a computer to execute: an acquisition step of acquiring concentrations of the biomarkers according to Embodiments 1 or 2 in blood collected from a subject to be tested;
a discriminant value calculation step of calculating a discriminant value which is a value of a multivariate discriminant based on a blood concentration of at least one biomarker of the blood concentrations of the biomarkers acquired in the acquisition step and the multivariate discriminant set in advance which has the blood concentration of the biomarker as a variable and has at least one biomarker as the variable; an evaluation step of evaluating the severity of depression of the subject to be tested based on the discriminant value calculated in the discriminant value calculation step; an an output step of outputting the obtained evaluation results.

6. The program for evaluating the severity of depression according to Embodiment 5, which causes a computer to further execute:
a step of causing a biomarker-measuring apparatus to measure the concentrations of the biomarkers in blood before the acquisition step.

7. A biomarker for predicting loss of interest/pleasure of a subject to be tested in at least any one of Patient Health Questionnaire (PHQ)-9, Beck Depression Inventory-II (BDI-2), and Hamilton Rating Scale for Depression (HAMD), the biomarker comprising:
at least one compound selected from the group consisting of acetylcarnitine, urocanic acid, 2-oxobutanoate, carbamoyl phosphate, proline, and 3-methylhistidine.

8. A method for predicting loss of interest/pleasure of a subject to be tested in at least any one of PHQ-9, BDI-2, and HAMD, the method comprising:
using a blood concentration of at least one compound selected from the group consisting of acetylcarnitine, urocanic acid, 2-oxobutanoate, carbamoyl phosphate, proline, and 3-methylhistidine of the subject to be tested.

9. A biomarker for predicting depressive feelings of a subject to be tested in at least any one of PHQ-9, BDI-2, and HAMD, the biomarker comprising:
at least one compound selected from the group consisting of N-acetylglutamate, 2-oxobutanoate, carnosine, 5-hydroxytryptophan, proline, and melatonin.

10. A method for predicting depressive feelings of a subject to be tested in at least any one of PHQ-9, BDI-2, and HAMD, the method comprising:
using a blood concentration of at least one compound selected from the group consisting of N-acetylglutamate, 2-oxobutanoate, carnosine, 5-hydroxytryptophan, proline, and melatonin of the subject to be tested.

11. A biomarker for predicting feelings of worthlessness/guilt of a subject to be tested in at least any one of PHQ-9, BDI-2, and HAMD, the biomarker comprising:
at least one compound selected from the group consisting of agmatine, adenosine triphosphate (ATP), argininosuccinate, tryptophan, valine, 5-hydroxytryptophan, proline, and phosphoenolpyruvate.

12. A method for predicting feelings of worthlessness/guilt of a subject to be tested in at least any one of PHQ-9, BDI-2, and HAMD, the method comprising:
using a blood concentration of at least one compound selected from the group consisting of agmatine, ATP, argininosuccinate, tryptophan, valine, 5-hydroxytryptophan, proline, and phosphoenolpyruvate of the subject to be tested.

13. A biomarker for predicting agitation/retardation of a subject to be tested in at least any one of PHQ-9, BDI-2, and HAMD, the biomarker comprising:

at least one compound selected from the group consisting of citrate, creatine, 5-hydroxytryptophan, 4-hydroxyproline, 3-hydroxybutyrate, fumarate, proline, and leucine.

14. A method for predicting agitation/retardation of a subject to be tested in at least any one of PHQ-9, BDI-2, and HAMD, the method comprising:

using a blood concentration of at least one compound selected from the group consisting of citrate, creatine, 5-hydroxytryptophan, 4-hydroxyproline, 3-hydroxybutyrate, fumarate, proline, and leucine of the subject to be tested.

15. A biomarker for predicting suicidal ideation of a subject to be tested in at least any one of PHQ-9, BDI-2, and HAMD, the biomarker comprising:

at least one compound selected from the group consisting of N-acetylglutamate, alanine, xanthurenate, xanthosine, kynurenine, kynurenate, citrate, 3-hydroxykynurenine, phenylalanine, and phosphoenolpyruvate.

16. A method for predicting suicidal ideation of a subject to be tested in at least any one of PHQ-9, BDI-2, and HAMD, the method comprising:

using a blood concentration of at least one compound selected from the group consisting of N-acetylglutamate, alanine, xanthurenate, xanthosine, kynurenine, kynurenate, citrate, 3-hydroxykynurenine, phenylalanine, and phosphoenolpyruvate of the subject to be tested.

17. A biomarker for predicting sleep-related disorder of a subject to be tested in at least any one of PHQ-9, BDI-2, and HAMD, the biomarker comprising:

at least one compound selected from the group consisting of agmatine, N-acetylaspartate, N-acetylglutamine, adenine, adenosine monophosphate (AMP), ATP, isocitrate, ornithine, carnitine, citrate, glucosamine, β-glycerophosphate, serotonin, tyrosine, threonine, pyruvate, pyroglutamate, phenylalanine, fumarate, pantothenate, 2-phosphoglycerate, 5-phosphoribosyl-1-pyrophosphate (PRPP), methionine, and 3-methylhistidine.

18. A method for predicting sleep-related disorder of a subject to be tested in at least any one of PHQ-9, BDI-2, and HAMD, the method comprising:

using a blood concentration of at least one compound selected from the group consisting of agmatine, N-acetylaspartate, N-acetylglutamine, adenine, AMP, ATP, isocitrate, ornithine, carnitine, citrate, glucosamine, β-glycerophosphate, serotonin, tyrosine, threonine, pyruvate, pyroglutamate, phenylalanine, fumarate, pantothenate, 2-phosphoglycerate, PRPP, methionine, and 3-methylhistidine.

19. A biomarker for predicting fatigue of a subject to be tested in at least any one of PHQ-9, BDI-2, and HAMD, the biomarker comprising: at least one compound selected from the group consisting of asparagine, N-acetylaspartate, 2-oxobutyrate, ornithine, β-glycerophosphate, melatonin, and proline.

20. A method for predicting fatigue of a subject to be tested in at least any one of PHQ-9, BDI-2, and HAMD, the method comprising: using a blood concentration of at least one compound selected from the group consisting of asparagine, N-acetylaspartate, 2-oxobutyrate, ornithine, β-glycerophosphate, melatonin, and proline.

**Claims**

1. A biomarker for use in predicting suicidal ideation of a subject to be tested in at least any one of Patient Health Questionnaire (PHQ)-9, Beck Depression Inventory-II (BDI-2), and Hamilton Rating Scale for Depression (HAMD), **characterized in that** the biomarker comprises at least one metabolite of the kynurenine pathway selected from the group consisting of xanthurenate, kynurenine, kynurenate, and 3-hydroxykynurenine.

2. The biomarker according to Claim 1,
wherein the biomarker further comprises at least one compound selected from the group consisting of N-acetylglutamate, alanine, xanthosine, citrate, phenylalanine, and phosphoenolpyruvate.

3. A method for collecting data for predicting suicidal ideation of a subject to be tested in at least any one of PHQ-9, BDI-2, and HAMD, comprising:

a step of measuring a concentration of a biomarker in a blood sample,
wherein the biomarker comprises at least one metabolite of the kynurenine pathway selected from the group consisting of xanthurenate, kynurenine, kynurenate, and 3-hydroxykynurenine.

4. The method according to Claim 3,
wherein the biomarker further comprises at least one compound selected from the group consisting of alanine, xanthosine, citrate, tryptophan and serine.

5. A program for predicting suicidal ideation of a subject to be tested in at least any one of PHQ-9, BDI-2, and HAMD, which causes a computer to execute:

> an acquisition step of acquiring concentrations of the biomarkers comprising at least one metabolite of the kynurenine pathway selected from the group consisting of xanthurenate, kynurenine, kynurenate, and 3-hydroxykynurenine in blood collected from a subject to be tested;
> a discriminant value calculation step of calculating a discriminant value based on a blood concentration of at least one biomarker of the biomarkers acquired in the acquisition step and the multivariate discriminant set in advance which has the blood concentration of the biomarker as a variable;
> an evaluation step of evaluating the severity of depression of the subject to be tested based on the discriminant value calculated in the discriminant value calculation step; and
> an output step of outputting the obtained evaluation results.

6. The program according to Claim 5, which causes a computer to further execute:
a step of causing a biomarker-measuring apparatus to measure the concentrations of the biomarkers in blood before the acquisition step.

7. The program according to Claim 5 or 6,
wherein the biomarkers further comprises at least one compound selected from the group consisting of alanine, xanthosine, citrate, tryptophan and serine.

FIG. 1

Data set-1
(Kyushu University)

Data set-2
(Osaka University)

Data set-3
(NCNP)

| 73 patients | 160 patients | 106 patients |

Excluded without
PHQ-9/HAMD-17

| 26 patients<br>Drug free | 24 patients<br>MOD<br>Drug medicated | 44 patients<br>29 MOD<br>15 BP |

Excluded
as outlier(s)

| 26 patients<br>Drug free | 25 patients<br>Drug free | 23 patients<br>MOD<br>Drug medicated | 41 patients<br>27 MOD<br>14 BP |

PHQ-9 Prediction
PLS model

HAMD-17 Prediction
PLS model

HAMD-17 Prediction
PLS model

HAMD-17 Prediction
PLS/MLR model

EP 3 677 914 A1

FIG. 2A

R$^2$=0.240
RMSEE=6.27
RMSEcv=7.87

FIG. 2B

R$^2$=0.263
RMSEE=6.38
RMSEcv=8.80

FIG. 2C

FIG. 2D

# FIG. 3

| Rank | Data set-1(PHQ-9) Drug free(N=26) | | Data set-1(HAMD-17) Drug free(N=25) | | Data set-2(HAMD-17) Medicated(N=23) | | Data set-3(HAMD-17) Mix(N=41) | |
|---|---|---|---|---|---|---|---|---|
| | Metabolite | VIP | Metabolite | VIP | Metabolite | VIP | Metabolite | VIP |
| 1 | 3HB | 6.11 | 3HB | 6.68 | 3HB | 6.58 | 3HB | 4.34 |
| 2 | Betaine | 4.43 | Creatine | 3.60 | Betaine | 4.12 | Isoleucine | 4.34 |
| 3 | Proline | 3.52 | Citrate | 3.49 | Carnitine | 3.21 | Betaine | 3.52 |
| 4 | Citrate | 3.37 | Betaine | 3.17 | Acetylcarnitine | 2.92 | Creatinine | 3.46 |
| 5 | Creatinine | 2.52 | Lysine | 2.44 | Creatinine | 2.55 | Phenylalanine | 3.35 |
| 6 | Acetylcarnitine | 2.45 | Proline | 2.18 | Creatine | 2.48 | Acetylcarnitine | 2.25 |
| 7 | Isoleucine | 1.66 | Glutamine | 2.06 | Ornithine | 2.35 | Citrate | 2.00 |
| 8 | Phenylalanine | 1.50 | Creatinine | 1.75 | Citrate | 1.50 | GABA | 1.92 |
| 9 | Glutamine | 1.48 | Carnitine | 1.68 | GABA | 1.47 | Dimethylglycine | 1.51 |
| 10 | Lysine | 1.29 | Phenylalanine | 1.57 | Isoleucine | 1.43 | Proline | 1.27 |
| 11 | Carnitine | 1.19 | Taurine | 1.36 | Arginine | 1.31 | Lysine | 1.19 |
| 12 | Creatine | 1.17 | TMAO | 1.15 | Norvaline | 1.29 | Argininosuccinate | 1.07 |
| 13 | GABA | 1.08 | GABA | 1.03 | TMAO | 1.11 | Kynurenine | 1.04 |

(3HB,3-Hydroxybutyrate;GABA, gamma-aminobutyric acid; TMAO,trimethylamine N-oxide)

The variable importance in projection (VIP > 1.0) denotes the degree of contribution to the PLS regression model.
Metabolites displayed in bold commonly contribute to the three independent PLS regression model.

EP 3 677 914 A1

# FIG. 4A

## Loss of interest/pleasure

| Metabolite | PHQ (1) | HAMD (2) |
|---|---|---|
| 2-Oxobutyrate | 0.368 | 0.283 |
| Acetyl carnitine | 0.293 | 0.204 |
| Proline | -0.365 | -0.155 |
| Carbamoylphosphate | 0.385 | 0.280 |
| 3-Methylhistidine | 0.224 | 0.224 |
| Urocanate | 0.203 | 0.249 |

## Agitation/retardation

| Metabolite | PHQ (8) | HAMD (16) | HAMD (17) |
|---|---|---|---|
| 5-Hydroxytryptophan | 0.337 | 0.626 | 0.629 |
| 4-Hydroxyproline | -0.382 | -0.290 | -0.250 |
| Creatine | -0.338 | -0.390 | -0.281 |
| Citrate | 0.187 | 0.432 | 0.388 |
| Leucine | -0.292 | -0.336 | -0.250 |
| Fumarate | 0.338 | 0.229 | 0.246 |

## Depressive feelings

| Metabolite | PHQ (2) | HAMD (1) |
|---|---|---|
| N-Acetylglutamate | -0.207 | -0.259 |
| 2-Oxobutyrate | 0.383 | 0.361 |

## Suicidal ideation (SI)

| Metabolite | PHQ (9) | HAMD (11) |
|---|---|---|
| Kynurenine | -0.289 | -0.284 |
| Xanthurenate | -0.325 | -0.201 |
| 3-Hydroxykynurenine | -0.226 | -0.256 |
| Kynurenate | -0.217 | -0.256 |
| Xanthosine | -0.244 | -0.250 |
| Citrate | 0.224 | 0.252 |
| Alanine | 0.229 | 0.290 |

## Feeling of worthlessness/guilty

| Metabolite | PHQ (6) | HAMD (10) |
|---|---|---|
| Proline | -0.082 | -0.301 |
| 5-Hydroxytryptophan | 0.196 | 0.324 |
| Phosphoenolpyruvate | 0.323 | 0.152 |
| ATP | -0.133 | -0.299 |
| Agmatine | -0.146 | -0.305 |

**FIG. 4B**

| SLEEP-RELATED METABOLITE | SLEEP DISORDER | CHANGES IN SLEEP | SLEEP-ONSET INSOMNIA | SLEEP MAINTENANCE INSOMNIA | EARLY MORNING AWAKENING INSOMNIA |
|---|---|---|---|---|---|
| Metabolite | PHQ9_3 | BDI_16 | SIGHD_06 | SIGHD_07 | SIGHD_08 |
| Threonine | 0.155 | 0.351 | 0.271 | 0.218 | 0.237 |
| Methionine | 0.093 | 0.258 | 0.471 | 0.068 | 0.273 |
| Pyruvate | 0.104 | 0.329 | 0.087 | 0.243 | 0.349 |
| Phenylalanine | 0.085 | 0.368 | 0.186 | 0.023 | 0.441 |
| 3Methylhistidine | 0.254 | 0.252 | 0.196 | 0.238 | 0.163 |
| Citrate | 0.080 | 0.430 | 0.331 | −0.080 | 0.271 |
| betaGlycerophosphate | 0.159 | 0.264 | −0.001 | 0.293 | 0.312 |
| Pantothenate | 0.113 | 0.239 | 0.222 | 0.213 | 0.224 |
| Tyrosine | 0.160 | 0.206 | 0.357 | 0.094 | 0.176 |
| 2-Phosphoglycerate | 0.017 | 0.375 | 0.327 | 0.096 | 0.138 |
| Fumarate | 0.342 | 0.225 | 0.021 | 0.293 | 0.063 |
| Isocitrate | −0.008 | 0.413 | 0.287 | −0.055 | 0.265 |
| AMP | 0.026 | 0.249 | 0.519 | −0.040 | 0.012 |
| Glucosamine | −0.017 | 0.347 | 0.165 | 0.000 | 0.259 |
| PRPP (Phosphoribosyl pyrophosphated) | −0.020 | 0.373 | −0.043 | 0.109 | 0.298 |
| N-Acetylglutamine | −0.053 | −0.365 | −0.513 | 0.112 | 0.007 |
| Ornithine | −0.034 | −0.454 | −0.424 | 0.121 | −0.029 |
| Adenine | −0.071 | 0.229 | −0.169 | −0.255 | −0.103 |
| Serotonin | −0.338 | −0.204 | 0.156 | −0.242 | −0.283 |
| Carnitine | −0.044 | −0.297 | −0.278 | 0.023 | −0.386 |
| Pyroglutamate | −0.304 | −0.270 | −0.214 | −0.057 | −0.159 |
| 2Phenylethylamine | −0.238 | −0.158 | −0.356 | −0.280 | −0.029 |
| ATP | −0.109 | −0.463 | −0.156 | −0.130 | −0.279 |
| Agmatine | 0.073 | −0.210 | −0.510 | −0.172 | −0.345 |
| N-acetylaspartate | −0.262 | −0.364 | −0.135 | −0.304 | −0.262 |

| FATIGUE-RELATED METABOLITE | FATIGUE | HYPODYNAMIA | FATIGUE | GENERAL SOMATIC SYMPTOM |
|---|---|---|---|---|
| Metabolite | PHQ9_4 | BDI_15 | BDI_20 | SIGHD_09 |
| betaGlycerophosphate | 0.153 | 0.223 | 0.303 | 0.313 |
| 2Oxobutyrate | 0.291 | 0.354 | 0.042 | 0.136 |
| Melatonine | 0.204 | 0.130 | 0.144 | 0.220 |
| Asparagine | −0.038 | −0.069 | −0.247 | −0.267 |
| Ornithine | −0.108 | −0.263 | −0.199 | −0.279 |
| Proline | −0.196 | −0.256 | −0.474 | 0.019 |
| N-acetylaspartate | −0.203 | −0.347 | −0.309 | −0.216 |

Legend: Positive / Negative

# FIG. 5

Legend:
— : Positive
∿∿∿ : Negative

FIG. 6

| Metabolite | Data set-1 | Data set-2 | Data set-3 |
|---|---|---|---|
| Citrate | 0.29 | 0.33 | 0.14 |
| Kynurenine | -0.28 | -0.30 | -0.32 |
| 3-Hydroxykynurenine | -0.26 | -0.25 | -0.10 |
| Kynurenate | -0.26 | -0.27 | 0.08 |

## FIG. 7A

## FIG. 7B

FIG. 8

Europäisches
Patentamt

European
Patent Office

Office européen
des brevets

# EUROPEAN SEARCH REPORT

Application Number

EP 20 15 8817

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ELIZABETH SUBLETTE M ET AL: "Plasma kynurenine levels are elevated in suicide attempters with major depressive disorder", BRAIN, BEHAVIOR AND IMMUNITY, ACADEMIC PRESS, SAN DIEGO, CA, US, vol. 25, no. 6, 9 May 2011 (2011-05-09), pages 1272-1278, XP028239160, ISSN: 0889-1591, DOI: 10.1016/J.BBI.2011.05.002 [retrieved on 2011-05-14] * the whole document * | 1-7 | INV. G01N33/68 G01N24/08 G01N33/49 H01J49/40 G16H50/20 G16H10/40 |
| X | L. BRUNDIN ET AL: "The role of inflammation in suicidal behaviour", ACTA PSYCHIATRICA SCANDINAVICA., vol. 132, no. 3, 8 August 2015 (2015-08-08), pages 192-203, XP055675152, DE ISSN: 0001-690X, DOI: 10.1111/acps.12458 * the whole document * | 1-7 | |
| X | EP 2 466 312 A1 (HUMAN METABOLOME TECHNOLOGIES INC [JP]; KAWAMURA NORIYUKI [JP]) 20 June 2012 (2012-06-20) | 1 | TECHNICAL FIELDS SEARCHED (IPC) G01N H01J G16H |
| Y | * the whole document * * paragraphs [0003], [0009] - [0049], [0067] - [0086]; claims 1-33; examples 1-3 * | 2-7 | |

-/--

The present search report has been drawn up for all claims

| Place of search | Date of completion of the search | Examiner |
|---|---|---|
| The Hague | 11 March 2020 | Boiangiu, Clara |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.......................................................................
& : member of the same patent family, corresponding
    document

EPO FORM 1503 03.82 (P04C01)

page 1 of 4

Europäisches
Patentamt

European
Patent Office

Office européen
des brevets

# EUROPEAN SEARCH REPORT

Application Number

EP 20 15 8817

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | REINHOLD G. LAESSLE ET AL: "Depression as a correlate of starvation in patients with eating disorders", BIOLOGICAL PSYCHIATRY, vol. 23, no. 7, 1 April 1988 (1988-04-01), pages 719-725, XP055593082, NEW YORK, NY; US ISSN: 0006-3223, DOI: 10.1016/0006-3223(88)90056-X * the whole document * * abstract * | 1-7 | |
| Y | LISA A. PAIGE ET AL: "A preliminary metabolomic analysis of older adults with and without depression", INTERNATIONAL JOURNAL OF GERIATRIC PSYCHIATRY, vol. 22, no. 5, 1 January 2007 (2007-01-01), pages 418-423, XP055593021, GB ISSN: 0885-6230, DOI: 10.1002/gps.1690 * the whole document * * abstract * | 1-7 | TECHNICAL FIELDS SEARCHED (IPC) |
| Y | MURPHY P ET AL: "The antidepressant properties of the ketogenic diet", BIOLOGICAL PSYCHI, ELSEVIER SCIENCE, NEW YORK, NY; US, vol. 56, no. 12, 15 December 2004 (2004-12-15), pages 981-983, XP004682245, ISSN: 0006-3223, DOI: 10.1016/J.BIOPSYCH.2004.09.019 * the whole document * | 1-7 | |

-/--

The present search report has been drawn up for all claims

| Place of search | Date of completion of the search | Examiner |
|---|---|---|
| The Hague | 11 March 2020 | Boiangiu, Clara |

Europäisches
Patentamt

European
Patent Office

Office européen
des brevets

# EUROPEAN SEARCH REPORT

Application Number

EP 20 15 8817

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | L. RANDALL ET AL: "Recurrent Diabetic Ketoacidosis in Inner-City Minority Patients: Behavioral, socioeconomic, and psychosocial factors", DIABETES CARE, vol. 34, no. 9, 20 July 2011 (2011-07-20), pages 1891-1896, XP055593087, US ISSN: 0149-5992, DOI: 10.2337/dc11-0701 * the whole document * * abstract * | 1-7 | |
| Y | WO 2015/030211 A1 (RIKEN [JP]; KURATSUNE HIROHIKO [JP] ET AL.) 5 March 2015 (2015-03-05) * the whole document * | 1-7 | |
| X,P | DAIKI SETOYAMA ET AL: "Plasma Metabolites Predict Severity of Depression and Suicidal Ideation in Psychiatric Patients-A Multicenter Pilot Analysis", PLOS ONE, vol. 11, no. 12, 16 December 2016 (2016-12-16), page e0165267, XP055592774, DOI: 10.1371/journal.pone.0165267 * the whole document * | 1-7 | **TECHNICAL FIELDS SEARCHED (IPC)** |
| A,P | CSILLA ARI ET AL: "Exogenous Ketone Supplements Reduce Anxiety-Related Behavior in Sprague-Dawley and Wistar Albino Glaxo/Rijswijk Rats", FRONTIERS IN MOLECULAR NEUROSCIENCE, vol. 9, 6 December 2016 (2016-12-06), XP055593098, DOI: 10.3389/fnmol.2016.00137 * the whole document * * abstract * | 1-7 | |

-/--

The present search report has been drawn up for all claims

| Place of search | Date of completion of the search | Examiner |
|---|---|---|
| The Hague | 11 March 2020 | Boiangiu, Clara |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

# EUROPEAN SEARCH REPORT

Application Number

EP 20 15 8817

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | SAMA SLEIMAN ET AL: "Exercise promotes the expression of brain derived neurotrophic factor (BDNF) through the action of the ketone body b-hydroxybutyrate", ELIFE, 2 June 2016 (2016-06-02), pages 1-21, XP055593073, * the whole document * | 1-7 | |
| A | WO 2010/025216 A1 (LUNDBECK & CO AS H [DK]; ANTONIJEVIC IRINA [US] ET AL.) 4 March 2010 (2010-03-04) * the whole document * | 1-7 | |

TECHNICAL FIELDS
SEARCHED (IPC)

The present search report has been drawn up for all claims

| Place of search | Date of completion of the search | Examiner |
|---|---|---|
| The Hague | 11 March 2020 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 15 8817

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2020

| Patent document cited in search report | | Publication date | Patent family member(s) | | Publication date |
|---|---|---|---|---|---|
| EP 2466312 | A1 | 20-06-2012 | CN | 102667485 A | 12-09-2012 |
| | | | CN | 104777314 A | 15-07-2015 |
| | | | DK | 2466312 T3 | 13-01-2020 |
| | | | EP | 2466312 A1 | 20-06-2012 |
| | | | JP | 5372213 B2 | 18-12-2013 |
| | | | JP | 5859502 B2 | 10-02-2016 |
| | | | JP | 2012211908 A | 01-11-2012 |
| | | | JP | 2014013257 A | 23-01-2014 |
| | | | JP | WO2011019072 A1 | 17-01-2013 |
| | | | US | 2012282592 A1 | 08-11-2012 |
| | | | US | 2015126623 A1 | 07-05-2015 |
| | | | WO | 2011019072 A1 | 17-02-2011 |
| WO 2015030211 | A1 | 05-03-2015 | JP | 6521321 B2 | 29-05-2019 |
| | | | JP | WO2015030211 A1 | 02-03-2017 |
| | | | WO | 2015030211 A1 | 05-03-2015 |
| WO 2010025216 | A1 | 04-03-2010 | AU | 2009285766 A1 | 04-03-2010 |
| | | | BR | PI0914859 A2 | 03-11-2015 |
| | | | CA | 2728171 A1 | 04-03-2010 |
| | | | CN | 102224256 A | 19-10-2011 |
| | | | EA | 201071324 A1 | 30-12-2011 |
| | | | EP | 2318551 A1 | 11-05-2011 |
| | | | JP | 2012501181 A | 19-01-2012 |
| | | | KR | 20110057188 A | 31-05-2011 |
| | | | US | 2011172501 A1 | 14-07-2011 |
| | | | WO | 2010025216 A1 | 04-03-2010 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62254185
- WO 2011019072 A
- JP 2014013257 A
- JP 5372213 B